# EUROPÄISCHE PATENTSCHRIFT

(11) **EP 3 688 503 B1**
(45) Veröffentlichungstag und Bekanntmachung des Hinweises auf die Patenterteilung: **04.03.2026**
(21) Anmeldenummer: 18785281.9
(22) Anmeldetag: 28.09.2018
(51) Int. Cl.: G02B 6/02, A61N 5/06, G02B 5/02

(54) **BELEUCHTUNGSSYSTEM MIT EINEM LICHTLEITER MIT DIFFUSOR-ELEMENT SOWIE VERFAHREN ZUM HERSTELLEN UND/ODER ZUM ZUMINDEST TEILWEISE ODER ABSCHNITTSWEISEN STRUKTURIEREN EINES DIFFUSOR- GRUNDKÖRPERS**
ILLUMINATION SYSTEM COMPRISING AN OPTICAL WAVEGUIDE WITH A DIFFUSER ELEMENT, AND METHOD FOR PRODUCING AND/OR AT LEAST STRUCTURING A DIFFUSER BASE BODY PARTIALLY OR IN SECTIONS
SYSTÈME D'ÉCLAIRAGE À GUIDE DE LUMIÈRE AVEC ÉLÉMENT DIFFUSEUR ET PROCÉDÉ DE FABRICATION ET/OU DE STRUCTURATION AU MOINS PARTIELLE OU PAR ENDROITS D'UN CORPS DE BASE DE DIFFUSEUR

(30) Priorität: 29.09.2017 DE 102017122756
(43) Veröffentlichungstag der Anmeldung: 05.08.2020
(73) Patentinhaber: SCHOTT AG, 55122 Mainz (DE)
(72) Erfinder: SCHULTHEIS, Bernd, 55270 Schwabenheim (DE); RUSSERT, Hubertus, 55270 Jugenheim (DE); KEIPER, Oliver, 65510 Hünstetten (DE); CRAMER, Martin, 65187 Wiesbaden (DE); MEINL, Jürgen, 65329 Hohenstein-Holzhausen (DE); WILMES, Lothar, 65375 Oestrich-Winkel (DE); GRIMM, Jonas, 65307 Bad Schwalbach (DE)
(74) Vertreter: Blumbach · Zinngrebe Patentanwälte PartG mbB
(86) Internationale Anmeldenummer: PCT/EP2018/076487
(87) Internationale Veröffentlichungsnummer: WO 2019/063799

(56) Entgegenhaltungen:
- EP-A1- 3 184 885
- WO-A1-93/25155
- DE-A1- 102012 208 810
- US-B1- 6 270 492

## Beschreibung

Die Erfindung betrifft ein Beleuchtungssystem insbesondere für ein medizintechnisches Therapie- und/oder Diagnosesystem sowie ein Verfahren zum Herstellen eines Diffusor-Grundkörpers, insbesondere für ein Beleuchtungssystem, und ein Verfahren zum zumindest teilweise oder abschnittsweisen Strukturieren, insbesondere zur Anpassung des Intensitätsverlaufs der seitlichen Abstrahlung, eines Diffusor-Grundkörpers,

Derartige Beleuchtungssysteme kommen verstärkt im medizinischen Umfeld zum Einsatz. Derzeit lassen sich folgende Anwendungsschwerpunkte klassifizieren:
- Photodynamische Therapie (PDT) zur Tumortherapie
- Endovenöse Lasertherapie (EVLT) zur Behandlung von Krampfadern
- Laser induzierte interstitielle Thermotherapie (LITT) sowie
- sonstige Anwendungen, u.a. im Bereich der Dentalmedizin, Augenheilkunde sowie Dermatologie.

Die Photodynamische Therapie (PDT) ist eine minimal-invasive Therapiemöglichkeit bei verschiedenen Krebserkrankungen. Unter der PDT versteht man ein Verfahren zur Behandlung von Tumoren und anderen Gewebeveränderungen (wie beispielsweise Gefäßneubildungen) mit Licht in Kombination mit einer lichtaktivierbaren Substanz. Zu Beginn der Behandlung werden den Patienten intravenös lichtsensible Substanzen, sogenannte Photosensitizer in die Blutbahn injiziert, die sich in beziehungsweise an den Krebszellen anreichern. Diese natürlichen Photosubstanzen konzentrieren sich in den Tumorzellen und bewirken dort eine starke Lichtempfindlichkeit. Dazu werden während der PDT-Behandlung in das Tumor-Gewebe mehrere Kanülen (typ. bis zu 8) gestochen, in die jeweils ein Lichtleiter mit einem Diffusor-Element eingeführt wird, wobei die Diffusor-Elemente möglichst räumlich über das Tumor-Gewebe verteilt angeordnet sein müssen. Laser-Licht, in der Regel mit Wellenlängen im sichtbaren Spektralbereich, zum Beispiel Grün-Licht mit 532 nm oder Rot-Licht mit 690 nm Wellenlänge, wird über die Lichtleiter in die Diffusor-Elemente eingekoppelt, so dass das Tumor-Gewebe möglichst gleichmäßig von innen ausgeleuchtet wird. Dabei bilden sich in diesen Zellen aggressive Sauerstoffradikale, welche die Tumorzellen selektiv zerstören. Im Gegensatz zu den kranken Zellen bleiben die gesunden Zellen von dieser chemischen Reaktion unberührt. Der genaue Wirkmechanismus ist u.a. in "Photodynamic Therapy of Cancer", Cancer Medicine, 2003 beschrieben.

Man unterscheidet hier zwischen Zylinder-Diffusoren mit typ. aktiven Längen von 10 bis 50 mm, Spot-Diffusoren, die einen vorwärts gerichteten Beleuchtungskegel erzeugen sowie Punktstrahler, die eine radiale Lichtemission aufweisen. Bei den Zylinder-Diffusoren kommt es im Betriebszustand insbesondere auf eine möglichst homogene seitliche Abstrahlung der Diffusor-Elemente über deren Länge an. Dies sowohl axial, d.h. an allen Punkten entlang jeder Linie vom proximalen zum distalen Ende in Richtung der Längsachse ist die Abstrahlungsintensität im Rahmen der Homogenitätsanforderung gleich, als auch radial, d.h. an allen Punkten jeder Umfangslinie entlang der Längsachse ist die Abstrahlungsintensität im Rahmen der Homogenitätsanforderung ebenfalls gleich, womit diese Diffusoren nahezu als Lambertsche Strahler wirken.

Gleichzeitig muss auch eine hohe Streueffizienz erzielt werden, um möglichst einen geringen Wärmeeintrag ins Gewebe sicher zu stellen. Typische Homogenitätsanforderungen an die seitliche Abstrahlung liegen bei maximal ± 10 bis 20 % Abweichung von der mittleren Intensität, wobei eine vorwärts gerichtete Abstrahlung, insbesondere aus dem distalen Ende heraus, von mehr als 10% des eingekoppelten Lichtes, typischerweise max. 5 %, zu vermeiden ist. Die typische Laser-Leistung liegt bei den PDT-Anwendungen bei < 5 W Dauerleistung, so dass pro cm Diffusorlänge max. zwischen 100 mW und 1000 mW, typischerweise zwischen 200 mW und 500 mW, abgestrahlt werden. Dies erlaubt derzeit den Einsatz von Kunststoff-basierten Diffusor-Ansätzen.

Bestehende Beispiele sind Diffusor-Elemente aus einem dünnen Silikon-Zylinder, in die Streupartikel in Form von Titanoxid-Nano-Partikel eingelagert sind. Die Schrift DE 10129029 A1 beschreibt eine flexible Vorrichtung zur thermischen Verödung von biologischem Gewebe mittels Laserstrahlung mit einem die Laserstrahlung führenden Lichtleiter, dessen distales Ende von einem für die Laserstrahlung transparenten Hüllschlauch umgeben ist, welcher das Faserende überragt und in seinem vor dem Faserende liegenden Volumen mit einer Silikonmatrix gefüllt ist, in die Streupartikel eingebettet sind, wobei in eine Kunststoffmatrix, vorzugsweise aus Silikon, nicht-streuende Partikel mit Durchmessern von wenigen Nanometern, vorzugsweise aus Siliziumdioxid, in einem Konzentrationsbereich von vorzugsweise 1-10% eingemischt sind und das distale Ende des Hüllschlauches durch ein für die Laserstrahlung transparentes oder opakes Endstück dicht verschlossen ist.

Diese können allerdings nur sehr aufwendig kostenintensiv mit ausreichender Abstrahlhomogenität hergestellt werden. Konglomerate der Streupartikel ergeben oft Abstrahlspots bei denen die Intensität dann deutlich über dem Durchschnitt liegt.

Diese Lichtleiter mit den Diffusor-Elementen werden in der Regel nur einmal verwendet und nach jeder Behandlung entsorgt. Daher besteht auch ein gewisser Kostendruck, was die Herstellkosten betrifft. Daher wird verstärkt auch über wiederverwendbare Lösungen nachgedacht. Derartige Lösungen müssen dann entsprechend den einschlägig bekannten Normen aufbereitbar, beispielsweise desinfizierbar und/ oder sterilisierbar sein. Als Aufbereitungsverfahren sind hier insbesondere Reinigungs-/ Desinfektionsverfahren mit stark basischen Lösungen und die Sterilisation mittels Autoklavieren bei Temperatur bis zu 135° C und typischen Dampfdrücken von etwa 3 bar zu nennen. Typischerweise geht man dann von einigen Zehn bis mehreren Hundert derartiger Aufbereitungszyklen aus. Dies erfordert hohe Ansprüche an die thermische-, chemische und auch hydrolytische Beständigkeit. Daher eignen sich dann insbesondere Lichtleiter- und Diffusor-Ansätze aus Glas- oder Quarzglasfasern.

Bei der EVLT führt der behandelnde Arzt über eine winzige Punktionsstelle einen Katheder in die betroffene Vene ein, der als Leitschiene für den Venenlaser dient. Durch gezielte seitliche Abstrahlung der Laserenergie mittels des Diffusors wird die Gefäßinnenwand anschließend stark erwärmt, wodurch die Vene kollabiert und verschlossen wird. Der krankhafte Rückfluss des venösen Blutes wird somit unterbunden. In der Folge verhärtet die Vene, bildet sich zurück und kann vom Körper abgebaut werden. Dabei werden derzeit in der Regel sogenannte Ring- oder Doppelring-Fire-Systeme als Abstrahlelement verwendet. Das Laserlicht wird in Form eines relativ scharf begrenzten Ring- oder Doppelringlichtes radial an das die Vene umschließende Gewebes abgegeben. Dabei wird der Lichtleiter mit dem Abstrahlelement zur gleichmäßigen Behandlung oft manuell mit möglichst konstanter Geschwindigkeit durch den zu behandelnden Venenabschnitt gezogen, was die Applikation erschwert, da bei Nichtbeachtung beziehungsweise zu langer Verweildauer an einer Stelle weitere Zellschädigungen entstehen können.

Vorteile würde hier ein Zylinder-Diffusor mit sich bringen, wie er bei PDT-Anwendungen zum Einsatz kommt. Allerdings sind bei der EVLT-Behandlung deutlich höhere Laser-Leistungen erforderlich. So beträgt die Laserleistung typisch zwischen 10 und 50 W bei Wellenlängen im NIR-Bereich, d.h. zwischen etwa 800 nm und 1480 nm, welche derzeit mit Dioden-Lasern (zum Beispiel 810 nm, 940 nm oder 1480 nm) oder Nd: YAG-Lasern (1064 nm) bereitgestellt wird. Inzwischen haben sich auch größere Wellenlängen um 2 µm zur EVLT-Behandlung etabliert. Zum Einsatz kommen dann beispielsweise Tm: YAG-Laser (1,9 µm) und Ho:YAG-Laser (2,1 µm). Aufgrund der Absorptionseigenschaften von Gewebe werden bei diesen Wellenlängen geringere Laserleistungen, typischerweise < 10 W, benötigt. Allerdings kommen hier bereits zwingend Quarzglas-Lichtleiter insbesondere für die Zuführung des Laserlichtes zum Einsatz.

Die Homogenitätsanforderungen an die seitliche Abstrahlung von Diffusoren, welche für EVLT eingesetzt werden können, sind gegenüber einer PDT-Anwendung weniger hoch und können bei maximal ±30 % bis maximal ± 50 % Abweichung von der mittleren Intensität betragen.

Bei der LITT handelt es sich um ein minimal-invasives Verfahren, das zur lokalen Tumordestruktion eingesetzt wird. Dabei wird unter bildgebender Kontrolle (zum Beispiel Sonographie/ MRT) der Tumor punktiert, eine (oder mehrere) Laserfaser(n) in den Tumorherd eingebracht und dieser durch thermische Energie verödet. Zum Einsatz kommen hier insbesondere Nd:YAG-Lasern (1064 nm) sowie Diffusor-Tip-Applikatoren. Die Laserleistung liegt bei etwa 5 bis 8 W (s. u. a. "Laserinduzierte Interstitielle Thermotherapie (LITT) bei malignen Tumoren", BÄK und KBV 01/2002).

Weitere Diffusor-Ansätze sind aus folgenden Schriften bekannt, wobei diese in vier Kategorien eingeteilt werden können: Volumenstreuende Diffusoren, Fasern mit aufgebrachten Streupartikeln, Diffusoren, die mittels Laserbearbeitung hergestellt werden und Diffusoren, die aus seitlich emittierenden Fasern gebildet sind.

Volumenstreuende Diffusoren sind beispielsweise in der Schrift EP 3184885A1 beschrieben. Diese beschreibt einen Diffusor am Ende einer Lichtleitfaser aus Quarzglas, wobei zur Erzeugung des Diffusors vorgesehen ist, auf dem distalen Faserende der Lichtleitfaser eine Streumasse aufzubringen und diese zu dem Diffusor zu verfestigen. Das Aufbringen der Streumasse umfasst die Schritte (a) Bereitstellen einer SiO₂-Körnung, die amorphe SiO₂-Teilchen enthält und die zu mindestens 90 Gew.-% aus SiO₂ besteht, (b) Bereitstellen eines Hohlköpers aus Glas mit einer Hohlraumwandung, die einen nach außen offenen Hohlraum umgibt, (c) Bilden einer Schüttung der SiO₂-Körnung in dem Hohlraum und Einbringen des Faserendes in den Hohlraum, so dass mindestens ein Teil des Faserendes in die Schüttung hineinragt, (d) thermisches Verdichten der Schüttung unter Ausbildung einer porenhaltigen und zu mindestens 90 Gew.-% aus SiO₂ bestehenden Sintermasse, die mindestens teilweise von einer Glashülle umgeben ist. Nachteilig bei derartigen Ansätzen ist, dass diese volumenstreuenden Ansätze einen stark exponentiellen Abfall der Intensität mit sich bringen. Auch sind poröse Materialien mit Blick auf deren Aufbereitbarkeit in medizintechnischen Anwendungen nicht bevorzugt.

In der Schrift US 6,810,184 B2 wird ein Ansatz beschrieben, bei dem nanoporöse Siliziumdioxid-plattierte optische Fasern verwendet werden, um Fasern mit integral gebildeten Diffusionspitzen und Diffusionspitzen herzustellen, die mit anderen Fasern verschmolzen werden können. Die offenbarten Diffusoren können zylindrisch hergestellt werden, wobei das Licht entlang seiner Länge diffundiert, sphärisch mit Licht, das nach außen in einem sphärischen Muster ausstrahlt, oder benutzerdefinierte, um unregelmäßige Flächen oder Volumina zu beleuchten. Gradienten- und Stufenindexeigenschaften können ebenfalls erreicht werden.

Die Schriften EP 2062077 A4, US 2009/0204111 A1 und DE 102015119875 A1 beschreiben Diffusoren, bei den zur Ihrer Erzeugung mittels Laser in beziehungsweise auf der Faser Strukturen ein- beziehungsweise aufgebracht werden.

Die Schrift EP 2062077 A4 beziehungsweise WO 2008/024397 A2 beschreibt u.a. einen Diffusor zum Ausgeben von optischer Energie hoher Leistungsdichte zu einer Behandlungsstelle am distalen Ende mindestens einer optischen Faser, wobei der Diffusor ein Abschnitt einer vorbestimmten Länge des distalen Endes mindestens einer optischen Faser ist, sowie Streuungszentren, die in dem Abschnitt der vorbestimmten Länge am distalen Ende der optischen Faser positioniert sind, wobei die Streuungszentren bewirken, dass ein Teil der eingegebenen optischen Energie radial auf eine Behandlungsstelle austritt. Dabei ist vorgesehen, dass die Streuungszentren in der vorbestimmten Länge des Faserkerns oder in oder nahe einer Grenzfläche zwischen dem Faserkern und der Umhüllung in der vorbestimmten Länge angeordnet sind. Die Streuungszentren sind Defekte des Faserkerns, wie beispielsweise Nanocracks oder Nano-Hohlräume, die lokalisierte Brechungsindex-Differentiale entweder in dem Kern oder in oder nahe der Grenzfläche zwischen dem Kern und der Umhüllung erzeugen. Die Streuungszentren können Streupartikel sein, die in dem Kern oder der Umhüllung des Kerns enthalten sind. Neben aufwendiger und schwer kontrollierbarer Einbringung bzgl. beispielsweise der Verteilung und/oder Größe der oben genannten Nanocracks oder Nano-Hohlräume, können diese sich auch negativ auf die Bruchanfälligkeit des Bauteiles auswirken. Des Weiteren ist bei allen Ansätzen entweder damit zu rechnen, dass, bei ausreichend homogener Ausgestaltung der Streuzentren, aufgrund des exponentiellen Abfalls der seitlichen Emission oder ungleichmäßiger Verteilungen eine seitliche Abstrahlung nicht mit geforderter Homogenität erzielt wird.

Die Schrift US 2009/0204111 A1 beschreibt ein Laser Delivery System mit einer optischen Faser mit (i) einem Kern und einer Mantelschicht, die mindestens einen Teil des Kerns bedeckt, wobei die Mantelschicht einen niedrigeren Brechungsindex als der Kern aufweist, und (ii) einen Nicht-Merkmalsabschnitt und einen Merkmalsabschnitt mit Merkmalen, die das Licht dazu zwingen, sich radial von dem Merkmalabschnitt auszutragen und ein gewünschtes radiales Lichtausgangsmuster zu schaffen. Dabei ist vorgesehen, dass die Merkmale aus der Gruppe ausgewählt sind, die aus spiralförmigen Strukturen, radialen Schnitten, axialen Schnitten und einer Kombination davon besteht.

Die DE 102015119875 A1 beschreibt einen Lichtwellenleiter umfassend einen lichtwellenführenden Kern, einen Bereich im Lichtwellenleiter, wobei in dem Bereich des Lichtwellenleiters Mikromodifikationen angeordnet sind und wobei die Anordnung der Mikromodifikationen geordnet ist. Das Verfahren zur Einbringung der Mikromodifikationen in Lichtwellenleitern umfaßt die Schritte (a) Fixierung eines Lichtwellenleiters in einer Halterung, wobei der Lichtwellenleiter und/ oder die Halterung beweglich gelagert sind, (b) Fokussieren von hochenergetischer Strahlung in eine Fokuslage, wobei die Fokuslage im Inneren des Lichtwellenleiters positionierbar ist, wobei die Strahlung von einer Strahlungsquelle im Pulsbetrieb erzeugt wird und wobei die Fokussiereinrichtung zum Fokussieren der hochenergetischen Strahlung beweglich gelagert ist und (c) Bewegen der Fokuslage durch den Lichtwellenleiter, wobei die Bewegung der Fokuslage im Inneren des Lichtwellenleiters in Abhängigkeit der Repetitionsrate gewählt wird.

Die Schrift DE 10 2012 208 810 A1 der Anmelderin beschreibt ein seitenemittierendes Glaselement, das eine Mehrzahl von an ihren Außenumfangsflächen unlösbar miteinander verbundenen Lichtleitelementen aus einem Glas mit dem Brechungsindex n₁ und zumindest ein Streuelement umfasst, das mit der Außenumfangsfläche zumindest eines Lichtleitelements unlösbar verbunden ist und das, wenn Licht in dem Glaselement geleitet wird, einen Anteil dieses Lichts seitlich aus dem Glaselement emittiert, wobei die einzelnen Lichtleitelemente von keinem Mantelglas mit einem von n₁ abweichenden Brechungsindex umhüllt sind und wobei zwischen den Lichtleitelementen eine Phasengrenze vorhanden ist. Diese Schrift beschreibt ebenfalls ein Verfahren zum Herstellen eines derartigen seitenemittierenden Glaselements, welches die Verfahrensschritte (i) Bereitstellen einer Mehrzahl von Lichtleitstäben aus einem Glas mit dem Brechungsindex n₁, (ii) Anordnen zumindest eines Streustabs aus einem Glas beinhaltend Streuzentren in oder bei der Mehrzahl der Lichtleitstäbe, so dass die Achsen der Lichtleitstäbe und des zumindest einen Streustabs zumindest weitestgehend parallel zueinander verlaufen und eine Vorform erhalten wird, (iii) Erwärmen der Vorform, (iv) Ausziehen der Vorform zu einem seitenemittierenden Glaselement, so dass sich die Außenumfangsflächen der Lichtleitstäbe unlösbar miteinander und mit dem zumindest einem Streustab verbinden. Die darin beschriebenen Ansätze werden insbesondere für dekorative Beleuchtungszwecke verwendet wobei hier insbesondere eine radial gerichtete Abstrahlwirkung erzielt werden soll.

Aufgabe der Erfindung ist es daher, eine kosteneffiziente Lösung für die Herstellung und Verwendung beziehungsweise mehrfache Verwendbarkeit von Diffusoren, insbesondere für Zylinder-Diffusoren sowie entsprechende Beleuchtungssysteme bereitzustellen. Weiterhin sollen diese die zuvor genannten Homogenitätsansprüche an die seitliche Abstrahlung bezogen auf die mittlere Intensität der seitlichen Abstrahlung und insgesamt möglichst Lambertschen Abstrahlungsverhalten insbesondere für PDT-Anwendungen einerseits und den Anforderungen hinsichtlich deren Verträglichkeit mit hohen Leistungsdichten insbesondere bei EVLT-Anwendungen anderseits gerecht werden. Wobei der, in der DE 10 2012 208 810 A1 beschriebene Ansatz eine Basistechnologie darstellt, bei welcher es vorteilhaft ist, diese hinsichtlich der Bereitstellung derartiger Diffusoren zu optimieren.

### Offenbarung der Erfindung:

Die Aufgabe der Erfindung wird durch die unabhängigen Ansprüche 1 und 27 gelöst, wobei vorteilhafte Weiterentwicklungen den unabhängigen Ansprüchen sowie der weiteren Offenbarung der Beschreibung und der Zeichnungen zu entnehmen sind.

Hierzu wird ein Beleuchtungssystem insbesondere für ein medizintechnisches Therapie- und/oder Diagnosesystem offenbart, umfassend wenigstens eine Laser-Lichtquelle, und einen Lichtleiter, der an einem proximalen Ende an die wenigstens eine Laser-Lichtquelle anschließbar und/ oder dieser zuordenbar ist, und welches am distalen Ende des Lichtleiters ein Diffusor-Element mit einer Längsachse aufweist, die senkrecht zu einer Einkoppelfläche des Lichtleiters in das oder in dem Diffusor-Element verläuft, wobei das Diffusor-Element im Betriebszustand Licht über seine aktive Länge seitlich zur Längsachse abstrahlt, wobei das Diffusor-Element mindestens einen Diffusor-Grundkörper aufweist und der Diffusor-Grundkörper mindestens ein Streuelement beinhaltet, wobei vorzugsweise das zumindest eine Streuelement entlang der Längsachse des Diffusor-Grundkörpers im Wesentlichen zu dieser parallel ausgerichtet ist oder in einem Winkel zur Längsachse des Diffusor-Grundkörpers angeordnet ist und am distalen Ende des Diffusor-Grundkörpers und/ oder dem Übergangsbereich zwischen Lichtleiter und Diffusor-Grundkörper und/ oder dem Diffusor-Grundkörper diesen zumindest teilweise oder abschnittsweise umschließende Einrichtungen zur Homogenisierung der Abstrahlungsintensität entlang der Längsachse des Diffusor-Grundkörpers vorgesehen sind, wobei das Beleuchtungssystem im Betriebszustand eine Intensitätsverteilung der seitlichen Abstrahlung aufweist, die um höchstens ± 50%, bevorzugt höchstens ± 30% und meist bevorzugt höchstens als ± 5% von der mittleren seitlichen Abstrahlungsintensität abweicht.

Als seitliche Abstrahlung wird im Rahmen der vorliegenden Offenbarung eine Abstrahlung verstanden, welche Richtungsanteile aufweist, die in radialer Richtung ausgehend von der Längsachse des Diffusor-Grundkörpers verlaufen. Als seitliche Abstrahlungsintensität wird die Intensität dieser Abstrahlung verstanden.

Diese Aufgabe kann insbesondere vorteilhaft auch dadurch gelöst, dass zumindest ein Streuelement entlang der kompletten Längsachse des Diffusor-Grundkörpers mit einheitlichem Querschnitt im Wesentlichen zu dieser parallel oder bei sich verjüngenden Diffusor-Grundkörpern in einem Winkel zur Längsachse angeordnet ist. Das zumindest eine Streuelement kann vorteilhaft auch rohrförmig und insbesondere koaxial zur Längsachse angeordnet sein. Eine Mehrzahl von Streuelementen kann in einer bestimmten vorgebbaren geometrischen Anordnung um die Längsachse des Diffusor-Grundkörpers angeordnet sein, bevorzugt in einer regelmäßigen Struktur um diese, insbesondere bevorzugt kreisförmig. Eine Mehrzahl von unter einem Winkel angeordneten Streuelementen trifft sich somit bevorzugt in einem Fluchtpunkt außerhalb des Diffusor-Grundkörpers.

Am distalen Ende des Diffusor-Grundkörpers, dem Übergangsbereich zwischen Lichtleiter und Diffusor-Grundkörper sind bevorzugt Einrichtungen und/ oder Maßnahmen zur Homogenisierung der seitlichen Abstrahlung entlang der Längsachse vorgesehen, die den Diffusor-Grundkörper zumindest teil- oder abschnittsweise und/ oder im Wesentlichen komplett umschließen. Beispielsweise gehören zu den Einrichtungen Hülsen, Hüllen, Kappen und/ oder Schichten am distalen Ende des Diffusors, um eine vorwärts gerichtete Abstrahlung aus dem distalen Ende zu verhindern beziehungsweise diese zurück zu reflektieren und damit den Streuprozessen im Diffusor-Grundkörper erneut bereitzustellen und andererseits Streulichteffekte und/oder Lichtreflexe am distalen Ende des Diffusor-Grundkörpers zu vermeiden.

Ähnliches gilt für den Übergangsbereich zwischen Lichtleiter und Diffusor-Grundkörper. Auch hier können Streulichteffekte und/oder Lichtreflexe auftreten, die durch entsprechend wirkende Elemente, beispielsweise Hülsen und/ oder Schichten an dieser Stelle unterdrückt werden können.

Der Lichtleiter kann eine Einzelfaser, beispielsweise Mono- oder Multimodelichtleitfaser umfassend einen Kern mit einem Kerndurchmesser und einen Mantel oder ein Faserbündel mit einem Faserbündeldurchmesser umfassen.

Damit lassen sich reproduzierbar und auch kostenoptimiert, im Betriebszustand homogen abstrahlende Diffusor-Elemente für medizinische Therapien, wie sie eingangs erwähnt sind, bereitstellen.

In einer bevorzugten Ausführungsvariante ist vorgesehen, dass die Streuelemente im Diffusor-Grundkörper radial gleichmäßig verteilt um die Längsachse des Diffusor-Grundkörpers angeordnet sind, wobei eine Kernzone um die Längsachse keine oder eine deutlich reduzierte Anzahl von Streuelementen je Flächeneinheit gegenüber der Anzahl von Streuelementen je Flächeneinheit außerhalb der Kernzone aufweist und somit die Streuelemente überwiegend außerhalb dieser Kernzone in der Matrix angeordnet sind. Damit kann erreicht werden, dass das eingekoppelte Licht, welches in der Regel mit geringer NA (< 0,3, typ. um die 0,2) eingekoppelt wird, nicht sofort an den Streuelementen gestreut wird. Andererseits kann durch die nahezu Streuelement-freie Kernzone genügend Licht ohne Streuung bis zum distalen Ende des Diffusor-Grundkörpers geleitet werden. Damit kann einerseits die Intensität nahe der Einkoppelstelle (proximales Ende des Diffusor-Grundkörpers) reduziert und andererseits die Intensität nahe dem distalen Ende Diffusor-Grundkörpers erhöht werden.

In einer weiteren bevorzugten Ausführungsvariante kann vorgesehen sein, dass der Diffusor-Grundkörper bezogen auf seine Querschnittsfläche eine Matrix aufweist, welche unterschiedliche Brechungsindices n₁ und n₁', insbesondere zwischen der Kernzone und dem Randbereich der Matrix, aufweist, in die die Streuelemente eingelagert sind. Damit kann bspw. die numerische Apertur NA in der Kernzone mit einem Matrix-Brechungsindex n₁ und außerhalb der Kernzone der Matrix mit einem Brechungsindex n₁' beeinflusst werden. Weiterhin kann damit die Ausbreitung des Lichtes im Diffusor-Grundkörper und somit eine Anregung der Streuzentren über die Länge des Diffusors an die erforderliche Abstrahlcharakteristik angepasst werden. Zudem kann beim Herstellprozess eine beliebige Querschnittsgeometrie der Kernzone mit dem Brechungsindex n₁, d.h. z.B. von im Wesentlichen kreisrunden bis hin zu einer polygonen oder sternförmigen Form realisiert werden.

Unterstützt werden kann die Homogenisierung der Intensität der seitlichen Abstrahlung, wenn der Durchmesser des Diffusor-Grundkörpers, in den die Streuelemente eingebettet sind, gleich groß oder größer ist als ein Kerndurchmesser oder Faserbündeldurchmesser des Lichtleiters.

Als besonders günstig hat sich ein Verhältnis zwischen Kerndurchmesser bzw. Faserbündeldurchmesser des Lichtleiters und Durchmesser der Matrix von ≤ 1,0 bis 0,7, besonders bevorzugt von ≤ 1,0 bis 0,8 herausgestellt.

Ein nur geringfügig gegenüber dem Durchmesser der Matrix kleinerer Kerndurchmesser bzw. Faserbündeldurchmesser kann dabei einen Intensitäts-Peak an der Einkoppelstelle (Übergangsbereich von Lichtleiter und Diffusor-Grundkörper) reduzieren.

Ein deutlich kleinerer Kerndurchmesser bzw. Faserbündeldurchmesser gegenüber dem Durchmesser der Matrix des Diffusor-Grundkörpers, das heißt ein Verhältnis von < 0,8, kann dagegen zu einer Intensitätsabsenkung an der Einkoppelstelle führen, die für bestimmte Anforderungen ebenfalls von Vorteil sein kann.

Liegt das Verhältnis zwischen 1 und 0,9, hat sich zudem herausgestellt, dass eine besonders robuste mechanische Kopplung bzw. Verbindung, zum Beispiel mittels Spleißen, zwischen Lichtleiter und Diffusor-Grundkörper erzielt werden kann.

Idealerweise weist das Diffusor-Element am distalen Ende des Diffusor-Grundkörpers eine Reflektorfläche in Form einer gerichtet reflektierenden Fläche beispielsweise, einer metallischen Spiegelfläche mit einer Metallbeschichtung insbesondere umfassend Al, Ag, Au oder einer diffus reflektierenden Fläche zum Beispiel umfassend eine weiße Farbschicht auf, welche das den Diffusor-Grundkörper passierte Licht in diesen zurück reflektiert. Damit kann der übliche exponentielle Abfall der Intensität des seitlich abgestrahlten Lichtes längs des Diffusor-Grundkörpers zumindest teilweise kompensiert beziehungsweise korrigiert werden. Die bereitstellbare Lichtmenge bei konstanter Streurate ist damit zumindest abschnittsweise verändert beziehungsweise anpassbar, so dass die seitliche Abstrahlung homogenisiert werden kann.

Als besonders wirksame Reflektoren, insbesondere auch zur Vermeidung von Hotspots, haben sich kurze, ca. 0,5 bis 2 mm lange, polierte, metallische Drahtabschnitte aus bspw. Aluminium oder Gold herausgestellt, die direkt in Kontakt mit dem Diffusor-Grundkörper gebracht sind und Wärmesenken bilden. Hotspots sind lokal erhöhte Lichtintensitäten, die bei Absorption beispielsweise an Grenzflächen zu unerwünschten lokalen Temperaturerhöhungen führen.

Weiterhin haben sich als besonders vorteilhaft aufgesputterte oder aufgedampfte dielektrische Reflektionsschichten auf dem distalen Ende des Diffusor-Grundkörpers herausgestellt, welche aus mehreren Schichten bestehen können und hinsichtlich der Reflektivität auf die Wellenlänge des verwendeten Lichtes abgestimmt werden können, dies bedeutet ein Maximum bei der Wellenlänge, auf welche abgestimmt wird, aufweisen können. Hiermit kann eine ideale Rückreflektion des im Betriebszustand eingekoppelten Lichtes beziehungsweise dessen Wellenlängen einerseits und eine Vermeidung von Hotspots andererseits erreicht werden.

Alternativ kann auch vorgesehen sein, den Reflektor aus einer breitbandig gut reflektierenden Silberschicht mit rückseitiger Passivierung auszuführen. Diese sind besonders robust und können störende Reflexe unterdrücken, die zu lokalen Intensitätsüberhöhungen und auch Hotspots führen können. Hiermit kann insbesondere ein sehr breitbandiger Reflektor realisiert werden, der sowohl im sichtbaren Spektralbereich (VIS) als auch im IR/MIR-Bereich, z.B. zwischen 1 µm und 2,5 µm Wellenlänge, sehr gute Reflektionseigenschaften aufweist. Eine rückseitige Passivierung verhindert eine Oxidation der Silberschicht.

Ist die Reflektorfläche konkav oder konvex ausgebildet, kann erreicht werden, dass reflektierte Strahlen zumindest teilweise mit nahezu parallelem Verlauf zur Längsachse und/ oder unter steilerem Winkel zur Längsachse zurückreflektiert und damit an den Streuelementen häufiger gestreut werden, so dass die Auskoppeleffizienz der seitlichen Abstrahlung zum distalen Ende des Diffusor-Elementes hin erhöht wird, was einen homogeneren Verlauf der Abstrahlintensität mit sich bringt.

Die Reflektorfläche kann auch als einseitig geschlossener Hohlkörper beziehungsweise eine Hülle oder Kappe mit einer in den Hohlraum oder in den transparenten Körper hinein reflektierenden Oberfläche ausgebildet sein. Dabei kann es sich um beispielsweise zylinderförmige Kunststoff-, Glas- oder Quarz-Kappen handeln, die sich an das distale Ende des Diffusor-Grundkörpers anschließen und die zumindest an einer deren Oberflächen wenigstens abschnittsweise gerichtet und/ oder diffus reflektierend, beispielsweise verspiegelt oder mit einer weißen Farbschicht versehen, ausgebildet sein können. Eine Verspiegelung kann als Reflektorfolie oder als reflektierende, beispielsweise aufgedampfte Beschichtung ausgeführt sein.

Vorteilhaft ist auch eine Metallkappe, die dann einen an das distale Ende des Diffusor-Grundkörpers anschließenden Hohlraum umschließt. Solcher Art gebildete Hohlräume können auch mit flüssigen, festen oder sich verfestigenden Materialien gefüllt vorliegen, womit beispielsweise Brechwerte eingestellt und/oder eine Verklebung der Kappe an das distale Ende realisiert werden kann.

Ebenso kann vorgesehen sein, dass die Kappen direkt, ohne Hohlraum das distale Ende abschließen. Besonders vorteilhaft umfassen diese Kappen dabei zumindest teil- oder abschnittsweise den Diffusor-Grundkörper radial über eine Länge von beispielsweise 0,5 bis 2 mm. Mit solchen Elementen können einerseits Streureflexe vermieden und andererseits bei gegebenenfalls vorhandenem Hohlraum die numerische Apertur der zurück reflektierten Strahlung in den Diffusor-Grundkörper über die Länge des Hohlraums beziehungsweise des transparenten Körpers eingestellt werden. Damit liegt wird die Reflektorfläche konkav oder konvex und/ oder als direkt oder beabstandet, einen Hohlraum zwischen Reflektorfläche und distalem Ende des Diffusor-Grundkörpers bildend, an den Diffusor-Grundkörper anschließender, Körper und/ oder Hülle, als einseitig geschlossener Hohlkörper, ausgebildet vor.

Metallkappen bieten zudem den Vorteil, dass diese als beispielsweise Röntgen-Marker genutzt werden können. Damit kann während eines Eingriffs beziehungsweise während der Therapie die genaue Position des Diffusor-Elementes im Gewebe des Patienten mittels Röntgenstrahlen-basierter Bildgebung visualisiert werden. Je nach bildgebenden Verfahren können entsprechend ausgeführte Kappen zumindest ähnlich wirken.

In bevorzugter Ausführungsform weist das Diffusor-Element zwischen dem proximalen Ende des Diffusor-Grundkörper und dem distalen Ende des Lichtleiters, eine Verbindungszone auf, die form- und/ oder stoffschlüssig mittels Verkleben, Spleißen oder Verpressen hergestellt ist und die zumindest den Durchmesser des Diffusor-Grundkörpers und den Kerndurchmesser oder den Faserbündeldurchmesser des Lichtleiters verbindet.

Zum Anpassen gegebenenfalls unterschiedlicher thermischer Ausdehnungskoeffizienten kann es von Vorteil sein, wenn zwischen dem proximalen Ende des Diffusor-Grundkörper und dem distalen Ende des Lichtleiters zusätzlich in der Verbindungszone ein Zwischenmedium vorgesehen ist. Dieses kann beispielsweise ein Übergangsglas oder Lotglas sein. Andererseits kann dies auch ein transparenter dauerelastischer Kleber sein. Des Weiteren kann in der Verbindungszone ein optisches Element angeordnet sein oder die Verbindungszone als optisches Element ausgeführt sein, um beispielsweise die Strahlführung und/oder Lichtlenkung durch geometrische oder Anpassung von Brechwerten zu modifizieren.

Zur Steigerung der mechanischen Stabilität insbesondere der Verbindungsstelle zwischen Diffusor-Element und Lichtleiter kann es von Vorteil sein, wenn die Verbindungszone mit einem Abdeckmaterial, beispielsweise einer Hülse oder einem Schlauch abgedeckt ist. Die Hülse oder der Schlauch kann dabei zumindest abschnittsweise transparent, transluzent und/oder opak und/ oder reflektierend aus Kunststoff, Glas, Keramik oder Metall, wie Neusilber, Titan oder Edelstahl oder Legierungen ausgebildet sein. Im einem Fall kann dies ein dünnwandiges Hüllglas sein, welches insbesondere die mechanische Stabilität erhöht. Metallhülsen bieten zudem den Vorteil, dass diese auch als beispielsweise Röntgen-Marker genutzt werden können. Damit kann während eines Eingriffs beziehungsweise während der Therapie die genaue Position des Diffusor-Elementes im Gewebe des Patienten visualisiert werden. Eine Variante sieht dabei vor, dass die Hülse aus einem starren Rohrabschnitt, beispielsweise aus TEFLON^{®}, Glas oder Quarz gebildet, und/ oder aus einem flexiblen Schlauch, zum Beispiel aus einem Silikon-Schlauch, gebildet ist. Enthält der Rohrabschnitt und/ oder der Schlauch zudem Streuzentren, kann die zuvor genannte Homogenisierung der Abstrahlcharakteristik weiter verbessert werden.

Eine besonders bevorzugte Ausführungsvariante für das Diffusor-Element schlägt vor, dass der Diffusor-Grundkörper distal mit der oben beschriebenen Reflektorfläche und deren Varianten sowie die Verbindungszone mit der oben beschriebenen Hülse und deren Varianten mit einer, diesen zumindest teilweise oder abschnittsweise umschließenden transparenten und/ oder transluzenten, eingefärbten oder farblosen Hülle versehen ist. Einerseits kann damit ein mechanischer Schutz erzielt werden. Andererseits kann durch geeignete Wahl der Materialien, insbesondere, wenn diese Streuzentren beinhalten, auch die Abstrahlcharakteristik hinsichtlich Homogenität der Intensität der seitlichen Abstrahlung weiter optimiert werden. Es kann damit beispielsweise eine Lambertsche Lichtabstrahlung unterstützt werden.

In einer bevorzugten Ausführungsvariante ist die Hülle zumindest abschnittsweise mit einem oder mehreren dünnwandigen Schrumpfschläuchen ausgeführt. Diese können zum einen auch eine zusätzliche diffuse Streuwirkung erzielen und somit eine Lambertsche Abstrahlung unterstützen. Zum anderen kann damit ein mechanischer Schutz erzielt werden und zum Beispiel mögliche Absplitterungen verhindern, falls der Diffusor beschädigt werden sollte. Als Hülle haben sich dafür beispielsweise ein dünnwandiger Schrumpfschlauch aus weiß eingefärbten PET mit einer Wandstärke von etwa 5 bis 15 µm erwiesen. Zur Unterdrückung von Reflexen kann zudem ein dünnwandiger, schwarz oder farbig eingefärbter Schrumpfschlauch partiell vorgesehen sein. Die Einfärbung kann derart gewählt werden, dass die Anwendungswellenlänge besonders gut absorbiert wird. Derartige Schrumpfschläuche sind zudem biokompatibel ausgeführt.

Um etwaige Unregelmäßigkeiten an den oder der Oberfläche der Hülle oder des Diffusor-Grundkörpers, wie bspw. Schmutz, Partikel, Rauigkeiten, die im Betriebszustand zu einer unerwünschten inhomogenen Abstrahlung führen können, auszugleichen, zu minimieren beziehungsweise zu verhindern, kann vorteilhaft zwischen und Hülle eine Immersionsflüßigkeit aufgebracht beziehungsweise eingebracht werden.

Der Diffusor-Grundkörper kann im Wesentlichen aus einer Matrix aus transparentem Kunststoff, Glas, Quarzglas oder Glaskeramik bestehen, wobei die darin eingelagerten Streuelemente beispielsweise bei einer Kunststoff-Matrix aus einem porösem oder pigmentierten oder zum Beispiel weiß eingefärbten Kunststoff, bei einer Glas-Matrix aus Poren, Partikeln, porösem oder pigmentierten oder zum Beispiel weiß eingefärbten oder Inhomogenitäten beinhaltendem Glas- oder Glaskeramik-Elementen und den darin umfassten Kristalliten, bei einer Quarz-Matrix aus Poren, porösem Quarzglas oder keramischen beziehungsweise polykristallinen Partikeln oder bei einer transparenten Glaskeramik-Matrixaus Poren, Partikeln, porösem oder pigmentierten oder zum Beispiel weiß eingefärbten oder Inhomogenitäten beinhaltendem Glas- oder Glaskeramik-Elementen und den darin umfassten Kristalliten bestehen können. Dabei können vorteilhaft auch Kombinationen der beispielhaft genannten Streuelemente in der jeweiligen Matrix vorliegen. Die Inhomogenitäten des Glases oder der Glaskeramik, die die Streuelemente bei Glas- oder Glaskeramikmatrixlösungen bilden können, umfassen beispielsweise Phasenseparationen, Entmischungen und/oder partikuläre Einlagerungen, Keime und/ oder Kristallite. Hierbei soll die Konzentrationen der Streuelemente im Streubereich von 10 ppm bis 1000 ppm und bevorzugt von 20 ppm bis 100 ppm betragen. Hierbei bezieht sich die Konzentrationsangabe in ppm auf den Anteil der Streupartikel im Verhältnis zu den Masseanteilen der Bestandteile des jeweiligen Materials, insbesondere den Kunststoff, die Glas-Matrix oder die Quarz-Matrix, in welchem die Streupartikel eingelagert sind. Hierbei weisen die jeweils gebildeten Streuelemente, diese bedeutet beispielsweise die Poren, Partikel, porösen oder pigmentierte oder zum Beispiel weiß eingefärbten oder Inhomogenitäten beinhaltenden Glas- oder Glaskeramik-Elemente und die darin umfassten Kristallite, bevorzugt einen Durchmesser von 10 nm bis 1000 nm auf, besonders bevorzugt von 100 nm bis 800 nm auf.

Ein Kunststoff-basierter Lösungsansatz für den Diffusor-Grundkörper aus Kunststoffstäben aus zum Beispiel PMMA, PET oder PC läßt sich bereits bei niedrigen Prozesstemperaturen bei dessen Herstellung oder Umformung realisieren. Allerdings weisen derart aufgebaute Diffusor-Grundkörper entsprechend eine eher niedrige thermische Beständigkeit auf, und sind daher eher für Anwendungen mit niedriger Laserleistung geeignet. Zudem eigenen sich diese nur für Anwendungen im sichtbaren Spektralbereich (VIS), da Kunststoffe eine in der Regel hohe Absorption im NIR- beziehungsweise IR-Bereich aufweisen. Glas-basierte Ansätze sind hier wesentlich robuster und vor allem thermisch stabiler, so dass auch größere Laserleistungen applizierbar sind. Als Elemente zum Aufbau des Diffusor-Grundkörpers kommen beispielsweise Stäbe der Gläser vom Typ N-BK7, optisches Bor-Kronglas der Anmelderin, Borosilikatglas oder Pb- beziehungsweise Schwermetall-freie in Betracht, wie sie unter anderem als Kernglas für optisch hochwertige Glasfasern für beispielsweise Endoskope oder Dentalstäbe zur Aushärtung von Zahnfüllungen zum Einsatz kommen. Mit letzteren können zukünftige RoHS-Anforderungen erfüllt werden. Derartige Gläser sind in der DE 10 2012 100 233 A1 sowie in der DE 10 2013 208 838 B4 der Anmelderin beschrieben.

Beispiele für solche Gläser für die Lichtleitstäbe beziehungsweise für die Matrix des Diffusor-Grundkörpers aus dem Bereich der bleifreien Zinn-Silikat-Gläser beziehungsweise Alkali-Zink-Silikat Gläser beinhalten folgende Komponenten (angegeben in Gew.% auf Oxidbasis):

| | von | bis |
|---|---|---|
| B₂O₃ | 0 | 24 |
| SiO₂ | 23 | 62,1 |
| Al₂O₃ | 0 | 10 |
| Li₂O | 0 | 10 |
| Na₂O | 0 | 18,5 |
| K₂O | 0 | 25,7 |
| BaO | 0 | 57,8 |
| ZnO | 0 | 40 |
| La₂O₃ | 0 | 25 |
| ZrO₂ | 0 | 10 |
| HfO₂ | 0 | 14,2 |
| SnO₂ | >0 | 2 |
| MgO | 0 | 8 |
| CaO | 0 | 8 |
| SrO | 0 | 24,4 |
| Ta₂O₅ | 0 | 22 |
| Y₂O₃ | 0 | 11,9 |
| Rb₂O | 0 | 15 |
| Cs₂O | 0 | 21 |
| GeO₂ | 0 | 7,5 |
| F | 0 | 2 |
| Σ R₂O | 5 | 20 |
| Σ MgO, CaO, SrO, ZnO | 20 | 42 |

Das Hüllrohr, welches um Lichtleitstab als späterer Mantel und/oder als Umhüllung der Vorform angeordnet sein kann, ist vorzugsweise aus einer der folgenden Gruppe 1 bis 4 aufgebaut, welches jeweils nachfolgende Komponenten beinhalten (angegeben in Gew.% auf Oxidbasis):

| | **Gruppe 1** | **Gruppe 2** | **Gruppe 3** | **Gruppe 4** |
|---|---|---|---|---|
| SiO₂ | 70 - 78 | 63 - 75 | 75 - 85 | 62 - 70 |
| Al₂O₃ | 5 - 10 | 1 - 7 | 1 - 5 | 1 - 10 |
| B₂O₃ | 5 - 14 | 0 - 3 | 10 - 14 | > 15 |
| Li₂O | 0 - 2 | 0 - 1 | 0 - 3 | 0 - 2 |
| Na₂O | 0 - 10 | 8 - 20 | 2 - 8 | 0 - 10 |
| K₂O | 0 - 10 | 0 - 6 | 0 - 1 | 0 - 10 |
| MgO | 0 - 1 | 0 - 5 | 0 | 0 - 5 |
| CaO | 0 - 2 | 1 - 9 | 0 | 0 - 5 |
| SrO | 0 - 1 | 0 | 0 | 0 - 5 |
| BaO | 0 - 4 | 0 - 5 | 0 | 0 - 5 |
| F | 0-1 | 0-1 | 0 | 0-1 |
| Cl | 0-1 | 0-1 | 0 | 0-1 |
| Fe₂O₃ | 0-2 | 0-2 | 0-2 | 0-2 |

Grundsätzlich kann auch ein Röntgen-opakes Glas oder eine entsprechende transparente Glaskeramik für den Diffusor-Grundkörper beziehungsweise für die eingelagerten Streuelemente und/ oder ein Hüllrohr eingesetzt werden. Dieses hat den Vorteil, dass der Diffusor-Grundkörper als Ganzes im Röntgenbild zumindest teilweise oder abschnittsweise erkennbar ist und somit die Position des Diffusors im Körper eines Patienten bestimmbar ist.

Im Hinblick auf Anwendungswellenlängen von 0,8 µm bis etwa 2,2 µm, zum Beispiel für die eingangs erwähnten EVLT-Anwendungen können auch spezielle IR-transparente Gläser, wie sie beispielsweise unten den Bezeichnungen N-PK52a, ein Phosphat-Kron-Glas, oder IRG7, ein Blei-Silikat-Glas, der Anmelderin bekannt sind, zum Einsatz kommen.

Bei einem Quarzglas-basierten Ansatz können insbesondere Anwendungen im UV und/ oder IR-Bereich bis etwa 2,5 µm Wellenlänge adressiert werden, wenn das Quarzglas besonders wenig OH-Gruppen aufweist. Als weiterer Vorteil ist hier die extrem hohe thermische Beständigkeit und die sehr niedrige Eigenabsorption von Quarz zu nennen, die insbesondere größere Laserleistung bis 50 W in der Anwendung erlaubt. Neben Streuelementen aus porösem Quarzglas können auch Streuelemente aus oder mit keramischen Pigmenten, zum Beispiel Titandioxid, Zirkonoxid oder Aluminiumoxid zum Einsatz kommen. Quarzglasbasierte Diffusor-Grundkörper lassen sich besonders gut an aus Quarzfasern gebildete Lichtleiter spleißen, wobei die Quarzfasern aus einem Kern und einem Mantel, oder auch Cladding genannt, bestehen, die sich geringfügig in deren Brechzahl unterscheiden. Das Cladding kann auch aus organischen Materialien, wie beispielsweise Fluor-Kunststoff, PMMA oder Polyimid, bestehen. Allerdings sind für den Herstellprozess der Diffusor-Grundkörper deutlich höhere Ziehtemperaturen erforderlich, als sie für Glas-basierte Ansätze benötigt werden.

Glaskeramik-basierte Ansätze für den Diffusor-Grundkörper und/oder die Streuelemente können aus einer transparenten Alumosilikat-Hochquarz-Mischkristall Glaskeramik gebildet sein, wie sie zum Beispiel für Kaminsichtscheiben oder Kochflächen Verwendung finden. Diese ist extrem thermoschockbeständig und besitzt eine hohe spektrale Transmission bis etwa 2,5 µm. Als Streuelemente eignet sich beispielsweise eine Keatit-Glaskeramik, die durch einen geeigneten Temper-Prozess aus der Hochquarz-Mischkristall-Glaskeramik hergestellt werden kann. Weiterhin eignen sich Cordieritglaskeramiken oder Magnesium-Aluminium-Silikat Glaskeramik als Diffusor-Grundkörper und/oder Streuelemente.

Ein hinsichtlich seines Herstellprozesses besonders bevorzugter Diffusor-Grundkörper ergibt sich, wenn der Diffusor-Grundkörper aus Lichtleitstäben aus Boro-Silikat-Glasstäben, Zinn-Silikat-Glasstäben oder Alkali-Zink-Silikat-Glasstäben und die Streuelemente aus Weißglas-Stäbchen gebildet sind, die mit einem Hüllrohr aus Borosilikatglas, Zinn-Silikat-Glas oder Alkali-Zink-Silikat-Glas umschlossen sind und die Vorform ausbilden.

In einer Weiterbildung der Erfindung können sowohl der Diffusor-Grundkörpers und das Hüllrohres aus gleichartigem Glas bestehen. Der Brechwert des Hüllrohes ist dabei bevorzugt nicht größer als der des Glases der Matrix, insbesondere bevorzugt sind beide Brechwerte gleichgroß. Dies begünstigt die Auskopplung des im Diffusor gestreuten Lichtes.

Damit sind kostengünstige Prozesse zur Herstellung von Diffusoren ermöglicht, die es erlauben diese in nahezu beliebiger Länge mit homogener Abstrahlintensität zu erhalten. Des Weiteren können diese auch hinsichtlich weiterer Geometrieparameter beispielsweise deren Durchmesser an eine Applikation angepaßt und als Halbzeuge verwendet werden, die anschließend beispielsweise für einen Anwendungsfall abgelängt und gegebenenfalls nachbehandelt werden können.

Zur Einhaltung eines vorgegebenen Toleranzbandes für den Intensitätsverlauf beziehungsweise Homogenität der im Betriebszustand seitlich abgestrahlten Strahlung über die Länge des Diffusor-Elementes beziehungsweise des Diffusor-Grundkörpers kann es erforderlich sein, dass der Diffusor-Grundkörper aus Abschnitten der im Ziehprozess auf den Durchmesser des Diffusor-Grundkörpers ausgezogenen Vorform gebildet ist, wobei der Diffusor-Grundkörper auch aus mehreren Abschnitten zusammengesetzt ausgebildet ist und die Abschnitte aus unterschiedlichen Vorformen mit unterschiedlicher Anzahl und Anordnung der Streuelemente hergestellt sind. Dabei können die Diffusor-Abschnitte mittels Spleißen oder Verkleben mit einem brechwertangepassten Kleber zu einem Diffusor-Grundkörper zusammengesetzt sein, wobei in einer Ausführungsform vorteilhaft die Streuwirkung des ersten Diffusor-Abschnitts, in dem die Lichteinkopplung erfolgt, am geringsten ausgebildet ist. In Richtung der Lichtausbreitung im Diffusor-Grundkörper nimmt dann bevorzugt in den einzelnen folgenden Diffusor-Abschnitten die Streuwirkung zu. Damit kann in Stufen die Intensität der im Betriebszustand seitlichen Abstrahlung innerhalb eines Toleranzbandes konstant gehalten werden.

Eine zumindest teilweise Kompensation des üblicherweise exponentiellen Intensitätsabfalls kann auch erreicht werden, wenn die Abschnitte der Diffusor-Grundkörper nach dem Ziehprozess einer Temperung, insbesondere einer Gradiententemperung, unterzogen werden. Mit dieser Gradiententemperung können die Streuelemente längs deren Längenausdehnung im Diffusor-Grundkörper hinsichtlich ihrer Streuwirkung nachträglich beeinflusst werden. So lässt sich beispielsweise ein Entmischungssprozess bei den beispielsweise als Streuelementen eingesetzten Weißglasstäbchen mit einer solchen Gradiententemperung variieren. Bei Glaskeramik-basierten Streuelementen kann die Kristallbildung und das Kristallwachstum respektive die Kristallitgröße und deren Verteilung entlang der Längsachse des Diffusor-Grundkörpers beeinflusst werden.

Um unerwünschte Streuung, Streulichteffekte und/oder Lichtreflexe insbesondere am Übergangsbereich von Lichtleiter und Diffusor-Grundkörper bzw. am distalen Ende des Diffusor-Grundkörpers zu reduzieren, kann in einer weiteren bevorzugten Ausführungsvariante vorgesehen sein, dass die Streuelemente am proximalen Ende in unmittelbarer Nähe zu einer Einkoppelfläche des Diffusor-Grundkörpers und/ oder am distalen Ende in unmittelbarer Nähe zur Reflektorfläche eine gegenüber der Streuwirkung entlang des Diffusor-Grundkörpers reduzierte Streuwirkung aufweisen. Dies kann beispielsweise durch eine zusätzliche Temperaturbeaufschlagung, zum Beispiel während des Spleißvorganges von proximalen Ende des Diffusor-Grundkörpers und Lichtleiter erzielt werden. Dadurch kann bspw. lokal eine bei als Streuelementen verwendeten Weißglasstäbchen vorhandene Entmischung (beispielsweise Phasenseparation, Entglasung) zumindest teilweise verändert, bspw. auch reduziert oder wieder rückgängig gemacht werden. Durch Letzteres nimmt die Streuwirkung in diesem Bereich ab. Ebenso kann das distale Ende des Diffusor-Grundkörpers erhitzt und dadurch beispielsweise durch konvex verformt und/ oder zumindest verrundet werden. Dabei kann ebenfalls die streuend wirkende Entglasung zumindest teilweise verändert und beispielsweise auch wieder rückgängig gemacht werden. Auch hier kann mit diesem zusätzlichen Temperaturprozeß eine Reduzierung einer unerwünschten Streuung, welche sich als Hotspots in diesem Bereich bemerkbar machen, erreicht werden.

Eine weitere zumindest teilweise Kompensation des Intensitätsabfalls des im Betriebszustand seitlich ausgekoppelten Lichtes kann auch erreicht werden, wenn der Diffusor-Grundkörper während des Ziehprozesses durch Variation, beispielsweise der Ziehgeschwindigkeit, -temperatur und/oder -kraft zumindest teilweise oder abschnittsweise kegelförmig ausgezogen ist und nach einem Ablängprozeß sich zumindest teilweise oder abschnittsweise verjüngende Diffusor-Grundkörper ergeben. Man erhält beispielsweise kegelförmig verjüngte Diffusor-Grundkörper, die aufgrund der Verjüngung eine Winkelaufweitung der Lichtstrahlen bewirken, so dass das Licht eine größere Wegstrecke zurücklegt und es daher in Längsrichtung des Diffusor-Grundkörpers zu einer stärkeren Streuung kommt. Die ansonsten parallel zur Längsachse angeordneten Streuelemente beim Herstellen der Vorform verlaufen im Bereich der Verjüngung nicht mehr parallel zur Längsachse des Diffusor-Grundkörpers, sondern in einem Winkel zu dieser und schneiden sich, idealerweise, in einem gemeinsamen Fluchtpunkt.

Darüber hinaus sind weitere nachgeschaltete Prozesse vorteilhaft durchführbar, bei denen der Intensitätsverlauf der seitlichen Abstrahlung des Diffusor-Grundkörper und/ oder des Diffusor-Elements korrigiert beziehungsweise angepasst werden kann.

Diese umfassen insbesondere Verfahren, die einerseits im Volumen und/ oder den Oberflächen eines Materials dessen Eigenschaften beispielsweise dessen Brechwert, und/oder Zusammensetzung, zum Beispiel als kolloidale Ausscheidungen und/ oder Keimbildung und/oder Kristallisation, zumindest lokal modifizieren können und/oder andererseits materialab- oder -auftragende Modifikationen in nahezu beliebiger geometrischer Form und Anordnung ermöglichen. Hierzu gehören beispielsweise Verfahren der Laserbearbeitung, die zum Beispiel mittels Kurzpuls- oder CO₂-Lasern das Einbringen von Brechwertveränderungen oder Erzeugen von Strukturen im Volumen, zum Beispiel Hohlräume, und/ oder den Oberflächen einbringen können.

Des Weiteren sind Druckprozesse zum Aufbringen beziehungsweise Herstellen von Strukturen, beispielsweise einer Rasterverlaufsstruktur auf der Oberfläche des Diffusor-Grundkörper und/ oder des Diffusor Elementes, zum Beispiel mittels druckbarer organischer oder keramischer Farben mit entsprechenden Pigmenten oder mittels einer Glasfluß-basierten Farbe, fallweise mit entsprechender thermischer Nachbehandlung, anwendbar. Ebenfalls durchführbarsind photolithografische Verfahren und Prozeßschritte, wie sie beispielsweise im Speziellen für eine Volumen- oder Oberflächenstrukturierung zum Beispiel photosensitiver oder photostrukturierbarer Gläser und Glaskeramiken eingesetzt werden. Ebenso möglich sind gegebenenfalls selektives naß- oder trockenchemisches Ätzen des Diffusor-Grundkörpers und/ oder des Diffusor-Elementes an deren Oberflächen, wobei auch hier photolithografische Prozeßschritte zur Anwendung kommen können. Auch mechanisch und/ oder abrasive wirkende Verfahren können zur Strukturierung, insbesondere Aufrauhung einer Oberfläche des Diffusor-Grundkörper und/ oder des Diffusor-Elementes, beispielsweise Schleifen, Läppen oder Sandstrahlen, eingesetzt werden.

Die vorgeschlagenen beispielhaften Methoden oder Verfahren können auch kombiniert angewandt werden. Die so erzeugbaren Diffusor-Elemente und/ oder der Diffusor-Grundkörper weisen somit zumindest teilweise oder abschnittsweise in deren Volumen und/ oder auf deren Oberflächen Strukturen auf.

In einer weiteren vorteilhaften Ausführungsvariante kann vorgesehen sein, dass der Diffusor-Grundkörper zumindest teil- und/oder abschnittsweise eine Beschichtung mit streuenden Partikeln und/ oder der Diffusor-Grundkörper zumindest teil- und/oder abschnittsweise eine zusätzliche, weitere Ummantelung aus einem eingefärbten Glas oder einem eingefärbten Kunststoff aufweist. Beispiele für eine derartige Beschichtung, welche zusätzliche eine Lambertsche-Abstrahlcharakteristik unterstützt und hierbei insbesondere eine in Lichteinkopplungsrichtung vorwärts gerichtete Abstrahlung reduziert, ist eine Beschichtung mit Bornitirid (BN). Weitere Beschichtungen dieser Art können zum Beispiel aus Titanoxid, Kalziumkarbonat oder Zirkonoxid bestehen.

Die zusätzliche Ummantelung kann bspw. als ein Weißglasrohr ausgeführt sein, welches in dessen Glasmatrix streuende Elemente enthält.

Im Übergangsbereich, im Bereich der Verbindungsstelle beziehungsweise im Bereich des Zwischenmediums zwischen Diffusor-Grundkörper und Lichtleiter kann als zusätzlich Ummantelung zum Beispiel ein Farbglasrohr vorgesehen sein, wobei die Einfärbung und deren Intensität so gewählt sein kann, dass insbesondere die verwendete Wellenlänge des Lichtes unterdrückt oder gar blockiert wird.

Damit können ungewollte Reflexe und damit eine ungewollte Seitenstrahlung unterdrückt werden. Entsprechende Ummantelungen aus Kunststoff sind beispielsweise eingefärbte Silikon- oder PTFE-Schläuche. Als weitere Variante kann insbesondere zur Unterdrückung einer vorwärts gerichteten Abstrahlung am distalen Ende des Diffusors vorgesehen sein, an den Diffusor-Grundkörper oder an dessen Hüll-Rohr ein eingefärbter, die Wellenlänge des Lichtes absorbierender Glasstab zu spleißen. Vorteilhaft sind auch entsprechende Tauchbeschichtungen aus Silikon oder entsprechenden Kunststoffen einsetzbar.

Die Herstellung eines erfindungsgemäßen Diffusor-Grundkörpers mit einer dem Anwendungszweck angepassten Beleuchtungsprofil, insbesondere der Homogenität der Intensität der im Betriebszustand seitlichen Abstrahlung, stellt ein schwerwiegendes Problem dar. Daher ist das Herstellungsverfahren eines erfindungsgemäßen Diffusor-Grundkörpers ebenfalls ein vorteilhafter weiterer Aspekt der Erfindung.

Es wird ein Verfahren angegeben zum Herstellen eines Diffusor-Grundkörpers, insbesondere für ein Beleuchtungssystem, nach einem der vorhergehenden Ansprüche, umfassend mindestens ein Streuelement, wobei vorzugsweise das zumindest eine Streuelement entlang der Längsachse eines Diffusor-Grundkörpers im Wesentlichen zu dieser parallel ausgerichtet ist oder in einem Winkel zur Längsachse des Diffusor-Grundkörpers angeordnet ist, beinhaltend die Verfahrensschritte
- Bereitstellen einer Mehrzahl von Lichtleitstäben aus einem Glas mit dem Brechungsindex n₁ und/ oder n₁',
- Anordnen der Mehrzahl der Lichtleitstäbe mit dem Brechungsindex n₁ und/ oder n₁' und zumindest eines Streustabs aus einem Glas oder einer Glaskeramik, die Streuzentren umfaßt, so dass die Längsachsen der Lichtleitstäbe und des zumindest einen Streustabs im Wesentlichen, parallel zueinander verlaufen und eine Vorform erhalten wird,
- Erwärmen der Vorform,
- Ausziehen der Vorform zu einem Diffusor-Grundkörper, so dass sich die Außenumfangsflächen der Lichtleitstäbe, unlösbar, formschlüssig miteinander und mit dem zumindest einem Streustab verbinden, insbesondere miteinander verschmelzen, und so die Matrix des Diffusor-Grundkörpers mit zumindest einem eingelagerten und/ oder an diesen angrenzendem Streuelement, gebildet aus dem zumindest einen ausgezogenen Streustab, bilden.

Es werden somit eine Mehrzahl von Lichtleitstäben aus einem Glas mit dem Brechungsindex n₁ bzw. n₁' bereitgestellt. Abhängig von dem zu erreichenden Beleuchtungsprofil werden zumindest einer oder mehrere Streustäbe aus einem Glas oder Glaskeramik beinhaltend die beschriebenen Streuzentren in einer erforderlichen Anzahl bereitgestellt und neben beziehungsweise zwischen den Lichtleitstäben angeordnet, so dass eine Anordnung von Lichtleitstäben und Streustäben entsteht, in der die Längsachsen von Lichtleitstäben und Streustäben vorteilhaft im Wesentlichen parallel zueinander angeordnet werden. Die Verteilung der Streustäbe in der Anordnung kann nach einem von dem gewünschten Beleuchtungsprofil abhängigen Muster erfolgen. Diese Anordnung wird durch geeignete Mittel fixiert und bildet so eine Vorform.

In einem folgenden Verfahrensschritt wird die Vorform erwärmt und zu einem seitlich abstrahlenden Glaselement so ausgezogen, so dass sich die Lichtleitstäbe und der zumindest eine Streustab an ihren Außenumfangsflächen unlösbar miteinander verbinden. Die Temperaturführung beim Ausziehen bewirkt auch, dass zwischen den Lichtleitelementen eine Phasengrenze vorhanden bleibt. Dies kann insbesondere dadurch erreicht werden, dass die Ziehtemperatur unterhalb der Schmelztemperatur des Glases der Lichtleitstäbe gehalten wird und diese insbesondere bei der Sintertemperatur zusammengesintert werden. Eine vollständige Verschmelzung der Lichtleitstäbe wird erfindungsgemäß vermieden. Ebenso durch die Temperatur-führung wird der bevorzugte Formschluss der Lichtleitstäbe und bedarfsweise auch der Streuelemente erreicht. Das so erhaltene Glaselement kann direkt den Diffusor-Grundkörper bilden. Insbesondere kann aber auch der Diffusor-Grundkörper und oder Abschnitte dessen durch Konfektionieren, beispielsweise Ablängen, des hergestellten Glaselementes erhalten werden. Die Matrix des Diffusor-Grundkörpers wird dabei aus den ausgezogenen, formschlüssig verbundenen Lichtleitstäben gebildet, in welche das zumindest eine Streuelement mit den Streuzentren, das aus den ausgezogenen Streustäben gebildet wird, ebenfalls formschlüssig, im Wesentlichen entsprechend dessen Anordnung in der Vorform, eingelagert ist.

In einer vorteilhaften Ausführungsform sind die Lichtleitstäbe wie beschrieben nicht vollständig miteinander verschmolzen, und auch der Streustab ist nicht vollständig mit zumindest einem der Lichtleitstäbe verschmolzen. Eine Phasengrenze kann dann auch zwischen dem Streustab und den Lichtleitstäben vorhanden sein und verbleibt somit auch innerhalb der gebildeten Matrix und den Streuelementen des Diffusor-Grundkörpers. Diese Ausführungsform lässt sich dadurch erreichen, dass die Erweichungstemperatur des Glases der Lichtleitstäbe gleich hoch oder niedriger als die Erweichungstemperatur der Streustäbe ist.

Eine ebenso vorteilhafte Ausführungsform sieht vor, dass die Lichtleitstäbe nicht vollständig miteinander verschmolzen und eine Phasengrenze zwischen diesen vorhanden ist, aber das zumindest eine Streuelement auf zumindest einen Lichtleitstab aufgeschmolzen ist. Dies kann erreicht werden, indem die Erweichungstemperatur des Glases der Streustäbe kleiner als die des Glases der Lichtleitstäbe gewählt wird. Vorteilhaft hat sich eine bis zu 50 K niedrigere Erweichungstemperatur des Glases der Streustäbe erwiesen, insbesondere eine bis zu 30 K niedrigere Erweichungstemperatur.

Beim Ausziehen werden aus den Lichtleitstäben die Matrix und aus den Streustäben die Streuelemente des Glaselements. Die Lichtleitstäbe bestehen daher aus einem Glas mit einem Brechungsindex n₁ und sind im Einzelnen nicht von einem Mantelglas mit dem Brechungsindex n₂ umhüllt.

Die Mittel zum Fixieren der Anordnung der Vorform aus Lichtleitstäben und Streustäben können beispielsweise Klammern sein, die nachher wieder entfernt werden. Bevorzugt wird allerdings ein Hüllrohr verwendet, das aus einem Glas mit dem Brechungsindex n₂ besteht. Die Anordnung der Lichtleitstäbe und Streustäbe wird in dieser Ausführungsform im Innern des Hüllrohres zusammengestellt. Besonders bevorzugt ist das Hüllrohr einseitig verschlossen. Das Hüllrohr umfängt die zuvor beschriebene Vorform zumindest entlang ihres Außenumfangs. Beim Erwärmen und Ausziehen erweicht das Hüllrohr und legt sich auf die Anordnung aus Lichtleitstäben und Streustäben, so dass ein Mantel um das Glaselement gebildet wird. Das durch das Erwärmen und Ausziehen erhaltene Produkt kann auch zerteilt und/oder weiterverarbeitet werden, um den Diffusor-Grundkörper zu erhalten.

Durch Variation der Parameter Geschwindigkeit, Temperatur und/ oder Kraft im Ziehprozess der Vorform können, gegebenenfalls nach einer Konfektionierung, zumindest teilweise oder abschnittsweise kegelförmige, sich verjüngende Diffusor-Grundkörper erhalten werden. Zumindest im Bereich einer Verjüngung verlaufen dann die Streuelemente nicht mehr parallel zur Längsachse des Diffusor-Grundkörpers, sondern in einem Winkel zu dieser.

Vorteilhaft wird auch ein Verfahren zum angegeben zum zumindest teilweise oder abschnittsweisen Strukturieren, insbesondere zur Anpassung des Intensitätsverlaufs der seitlichen Abstrahlung, eines Diffusor-Grundkörpers umfassend mindestens ein Streuelement, wobei vorzugsweise das zumindest eine Streuelement entlang der Längsachse des Diffusor-Grundkörpers im Wesentlichen zu dieser parallel ausgerichtet ist oder in einem Winkel zur Längsachse des Diffusor-Grundkörpers angeordnet ist, und/ oder
eines Diffusor-Elementes, wobei
der Diffusor-Grundkörper mit der Reflektorfläche und der Verbindungszone zumindest teilweise oder abschnittsweise mit einer transparenten oder transluzenten Hülle umschlossen ist und das Diffusor-Element bildet; bevorzugt ist die Hülle aus einem starren Rohrabschnitt und/ oder aus einem flexiblen Schlauch gebildet, bevorzugt enthält der Rohrabschnitt und/ oder der Schlauch Streuzentren, die im Volumen und/ oder den Oberflächen dessen Eigenschaften und/oder Zusammensetzung zumindest lokal modifizieren und/oder materialab- oder auftragend Strukturen in nahezu beliebiger geometrischer Form und Anordnung in und/oder auf diesen ausbilden,
umfassend
   - Verfahren der Laserbearbeitung, insbesondere mittels Kurzpuls- oder CO₂-Lasern, die bevorzugt Brechwert- und/ oder Zusammensetzungsänderung oder Erzeugen von Strukturen im Volumen und/ oder den Oberflächen einbringen
   - Druckprozesse zum Aufbringen beziehungsweise Herstellen insbesondere einer Rasterverlaufsstruktur mittels druckbarer organischer oder keramischer Farben mit entsprechenden Pigmenten oder mittels einer Glasfluss-basierten Farben
   - Verfahren des nasschemischen oder trockenchemischen Ätzens,
   - fotolithografische Verfahren,
   - abrasive, mechanische Bearbeitungsverfahren oder
eine Kombination dieser Verfahren.

Eine bevorzugte Verwendung des Beleuchtungssystems, wie es zuvor in seinen verschiedenen Ausführungsvarianten beschrieben wurde, sieht den Einsatz für eine photodynamische Therapie (PDT) beispielsweise zur Tumortherapie, für eine endovenöse Lasertherapie (EVLT) beispielsweise zur Behandlung von Krampfadern, für eine Laser-induzierte interstitielle Thermotherapie (LITT) oder für Anwendungen im Bereich der Dentalmedizin, Augenheilkunde sowie Dermatologie vor, wie diese eingangs beschrieben wurden. Im Bereich Dentalmedizin sind hier insbesondere Applikationen zur Wund- oder Parodontose-Behandlung zu nennen. Darüber hinaus gibt es Anwendungen in der Hirnforschung, bei denen mittels Licht einzelnen Gehirnbereich stimuliert und damit Krankheitssymptome behandelt werden können.

Eine weitere Verwendung des Beleuchtungssystems, wie es zuvor in seinen verschiedenen Ausführungsvarianten beschrieben wurde, sieht den Einsatz für eine photodynamische Therapie (PDT) zur Tumortherapie vor, wobei zumindest ein Lichtleiter mit dem Diffusor-Element aus anderen Diffusor-Elementen abgestrahltes Licht aufnimmt und über den Lichtleiter einem Detektor zur spektroskopischen Analyse weiterleitet. Dabei werden dem Patienten neben den verschiedenen Lichtaussendenden Diffusor-Lichtleitern auch lichtempfangende Diffusor-Lichtleiter appliziert, wobei anhand der spektralen Unterschiede zwischen eingekoppeltem und empfangenen Licht auf ein Ansprechen der PDT-Behandlung geschlossen werden kann (siehe dazu Finlay et al., Proc. SPIE Int. Soc. Opt. Eng. 2014, June 14; 5315: Page 132-142).

Darüber hinaus sind auch Anwendungen im industriellen Bereich vorteilhaft, etwa zur Inspektion von schwer zugänglichen Stellen beispielsweise an oder in einer Maschine, bei denen es insbesondere auf eine homogene Ausleuchtung ankommt, oder auch spektroskopische Anwendungen oder in der Biochemie, bei der durch Licht biochemische In-Vitro-Reaktionen stimuliert werden.

Die Erfindung wird im Folgenden anhand eines in den Figuren dargestellten Ausführungsbeispiels näher erläutert. Es zeigen:
Fig. 1 schematisch ein Beleuchtungssystem mit einem Lichtleiter und einem Diffusor-Element in einer PDT-Anwendung,
Fig. 2 in eine schematische Querschnitt-Darstellung des Diffusor-Elements,
Fig. 3a bis 3d verschiedene Ausführungsbeispiele für die Anordnung von Streuelementen in einem Diffusor-Grundkörper,
Fig. 4a und 4b verschiedene Ausführungsbeispiele für Streuelemente in einer Matrix des Diffusor-Grundkörpers,
Fig. 5a bis 5c schematisch verschiedene Ausgestaltungsbeispiele einer Reflektorfläche des Diffusor-Grundkörpers,
Fig. 6a bis 6c schematisch verschiedene Ansätze zur Homogenisierung eines Intensitätsverlaufs,
Fig. 7 in einem Verlaufsdiagram verschiedene schematische Intensitätsverläufe und
Fig. 8 in einem weiteren Verlaufsdiagram die gemessenen Intensitätsverläufe.
Fig. 9 zeigt eine Aufnahme des Querschnitts einer angeschliffenen Fläche eines Diffusor-Grundköpers nach Fig. 3a
Fig. 10 zeigt eine Aufnahme des Querschnitts einer nur geritzten und gebrochenen Fläche eines Diffusor-Grundköpers nach Fig. 3d
Fig. 11 einen Querschnitt durch einen Diffusor-Grundkörper 43, bei dem dessen Matrix 43.4 unterschiedliche Brechungsindices n₁ und n₁' aufweist,
Fig. 12 einen Ausschnitt aus der schematischen Querschnitt-Darstellung des Diffusor-Elements der Fig. 2.

### Detaillierte Beschreibung bevorzugter Ausführungsformen

Bei der nachfolgenden Beschreibung der detaillierten Ausführungsformen bezeichnen gleiche Bezugszeichen in den beiliegenden Figuren jeweils gleiche oder gleich wirkende Bestandteile.

Zum besseren Verständnis werden die nachfolgenden Definitionen vorgenommen.

Im Sinne der vorliegenden Offenbarung umfasst der Begriff des Beleuchtungssystems Beleuchtungsvorrichtungen und insbesondere Beleuchtungsvorrichtungen, welche zur Anwendung für medizintechnische Zwecke geeignet und insbesondere, soweit diese mit lebendem Gewebe in Kontakt treten sollen, zumindest abschnittsweise desinfizierbar und/ oder sterilisierbar sind.

Die Aussage "für ein medizintechnisches Therapie- und/oder Diagnosesystem" umfasst auch die Verwendung oder Anwendung des hier offenbarten Beleuchtungssystems selbst als medizinisches Therapie- und/oder Diagnosesystem.

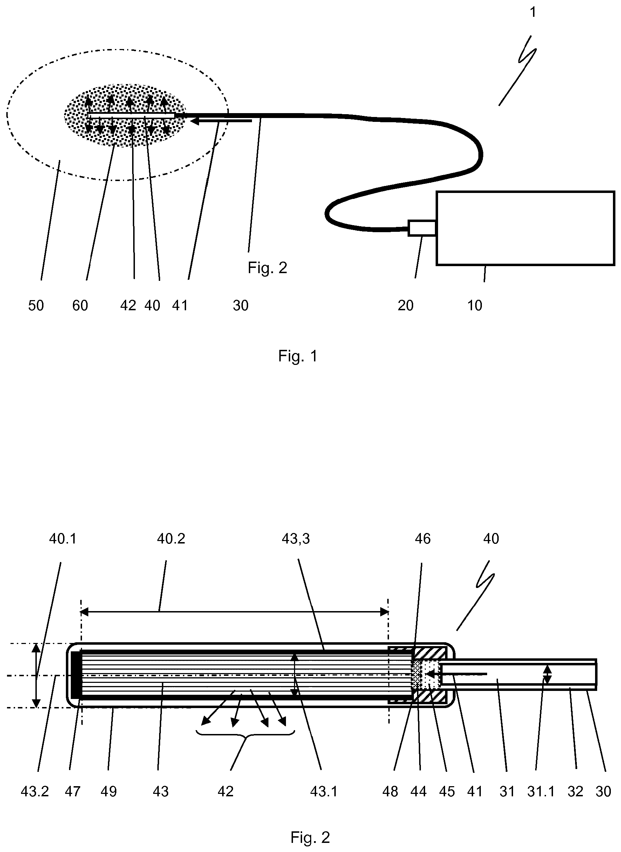

Fig. 1 zeigt schematisch den Aufbau eines Beleuchtungssystems 1 gemäß der einer bevorzugten Ausführungsform der Erfindung. Beispielhaft ist hier eine medizintechnische PDT-Anwendung dargestellt.

Im gezeigten Beispiel umfasst das Beleuchtungssystem 1 aus eine Laser-Lichtquelle 10, welche im Betriebszustand Licht in einem bestimmten Spektralbereich aussendet. Für PDT-Anwendungen, wie sie eingangs beschrieben sind, werden Laser verwendet, die auf den zuvor verabreichten biochemisch modifizierten Farbstoff (Photosensitizer) abgestimmte Wellenlänge üblicherweise im sichtbaren Bereich, beispielsweise im grünen Spektralbereich bei 532 nm oder im roten Spektralbereich bei zum Beispiel 690 nm, aussenden.

Ein Lichtleiter 30 ist mit einem Stecker 20 an seinem proximalen Ende an die Laser-Lichtquelle 10 angeschlossen. Als proximales Ende wird hierbei das Ende des Lichtleiters 30 bezeichnet, in welches Licht eingekoppelt wird. Am distalen Ende weist der Lichtleiter 30 ein Diffusor-Element 40 auf, welches über hier nicht dargestellte Kanülen in ein Tumor-Gewebe 60 eingebracht wird, welches sich innerhalb eines gesunden Gewebes 50 gebildet hat. Als distales Ende wird hierbei das andere Ende des Lichtleiters 30 bezeichnet, welches in der Regel zu dem proximalen Ende des Lichtleiters 30 entfernt angeordnet ist und aus welchem insbesondere Licht austritt.

Die Laserstrahlung gelangt dabei über eine Lichteinkopplung 41 am Diffusor-Element 40 in das Diffusor-Element 40 und wird über die Länge des Diffusors seitlich abgestrahlt (Lichtauskopplung 42). Dabei kommt es auf eine möglichst homogene Abstrahlung über die Länge des Diffusor-Elementes 40 an. Insbesondere sind Intensitätsspitzen zu vermeiden. Durch eine photoinduzierte biochemische Reaktion, wie sie eingangs beschrieben ist, kommt es nach der Behandlung idealerweise zu einem Absterben des Tumor-Gewebes 60. In der Regel werden als Lichtleiter 30 Quarz-Fasern verwendet, wobei die Stecker 20 in der Regel als koaxialer Steckverbinder, sogenannte SMA-Stecker, ausgebildet sind, bei denen die Fasern in den Stecker 20 geklebt sind. Vorteilhaft hinsichtlich der thermischen Belastbarkeit können auch Stecker 20 mit Neusilber-Hülsen sein, bei denen der Lichtleiter 30 in die Neusilber-Hülse formschlüssig durch plastische Verformung eingebracht, gecrimpt, ist. Darüber hinaus können bei größeren Laser-Leistungen auch Stecker 20 zum Einsatz kommen, bei denen das Faserende des Lichtleiters 30 durch ein Kegelprisma geschützt ist, was vorteilhaft bei Fehljustagen sein kann.

Fig. 2 zeigt schematisch den Aufbau eines Diffusor-Elementes 40 gemäß einer bevorzugten Ausführungsform der Erfindung.

Das Diffusor-Element 40 besteht aus einem Diffusor-Grundkörper 43, welcher über eine Verbindungszone 44 an den Lichtleiter 30 befestigt ist. Der Lichtleiter 30 besteht bei den zuvor beschriebenen Anwendungen meist aus Quarzglas mit einem Kern 31 mit einem Brechungsindex n₁ und einem Kerndurchmesser 31.1 von üblicherweise zwischen 200 und 600 µm sowie einem Mantel 32 mit einem Brechungsindex n₂, wobei gilt n₁ > n₂. Die damit üblicherweise erzielbare numerische Apertur NA beträgt etwa 0,22. Die Lichteinkopplung 41 erfolgt über Einkoppelfläche 46 des Diffusor-Grundkörpers 43.

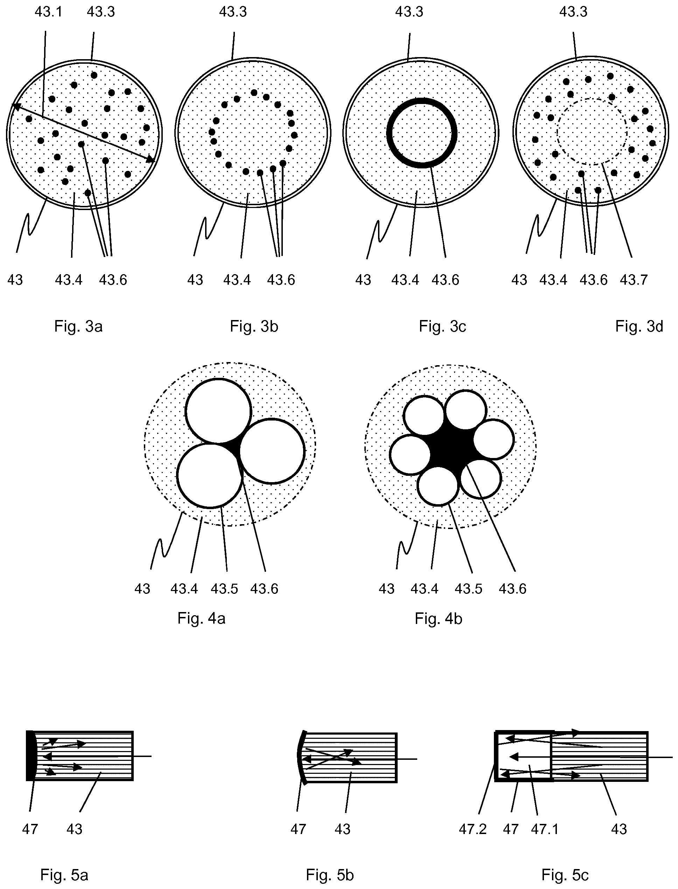

Der Diffusor-Grundkörper 43 mit seinem Durchmesser 43.1 umfasst bei einer bevorzugten Ausführungsform ein Hüllrohr 43.3 und eine Matrix 43.4 aus Matrix-Elementen 43.5 mit eingelagerten Streuelementen 43.6 oder besteht aus einem Hüllrohr 43.3 und einer Matrix 43.4 aus Matrix-Elementen 43.5 mit eingelagerten Streuelementen 43.6 (hier nicht dargestellt, siehe dazu Fig. 3a bis 3d sowie Fig. 4a und 4b). Um im Betriebszustand die Homogenitätsanforderungen hinsichtlich der Intensität der seitlichen Abstrahlung erfüllen zu können, umfasst der Diffusor-Grundkörper 43 in einer Ausführungsform zumindest 10, bevorzugt zumindest 20 eingelagerte Streuelemente 43.6, ebenso bevorzugt nicht mehr als 100, da sich sonst die Zusammenstellung der Vorform zu aufwendig darstellt.

Das Verhältnis der Querschnittsflächen von eingelagerten Streuelementen 43.6 und Diffusor-Grundkörper 43 ergibt sich zu ≤ 0,015, bevorzugt ≤ 0,005, besonders bevorzugt ≤ 0,002. Die Streuelemente 43.6 sind dabei über die komplette Länge des Diffusor-Grundkörpers 43 im Wesentlichen parallel zur Längsachse 43.2 ausgerichtet.

In vorteilhafter Ausgestaltung ist der Durchmesser des Diffusor-Grundkörper 43 grösser als der Kerndurchmesser 31.1 oder Faserbündeldurchmesser 31.1 des Lichtleiters 30 ausgelegt, so dass einerseits kein unkontrolliertes Streulicht beispielsweise in das Hüllrohr 43.3 eingekoppelt wird. Andererseits können damit Montage und die Justage von Lichtleiter 30 und Diffusor-Grundkörper 43 erleichtert und/oder Montagetoleranzen ausgeglichen werden.

Das Verhältnis des Kerndurchmessers 31.1 oder Faserbündeldurchmessers 31.1 des Lichtleiters 30 und des Durchmessers des Diffusor-Grundkörpers 43.1 mit den eingelagerten Streuelementen 43.6 beträgt damit vorteilhaft ≤ 1,0, bevorzugt zwischen 1,0 und 0,8. Je nach gewünschter Abstrahlcharakteristik kann auch Verhältnis von ≤ 0,8 vorgesehen sein.

In der Verbindungszone 44 zwischen dem proximalen Ende des Diffusor-Grundkörpers 43 und dem distalen Ende des Lichtleiter 30 kann ein optisches Element angeordnet sein, das beispielsweise als Strahlformungselement, Lichtleitelement oder faseroptischer Taper, gegebenenfalls konisch ausgebildet sein kann. So werden auch geometrische Anpassung beispielsweise von Durchmesserunterschieden ermöglicht. Hierbei bezeichnet das proximale Ende des Diffusor-Grundkörpers 43 das Ende des Diffusor-Grundkörpers 43, in welches Licht eingekoppelt wird.

Zur Vermeidung von Streulicht aus der Verbindungszone 44 aber auch als mechanische Stabilisierung der Verbindungszone 44 ist eine Hülse 48 aus Kunststoff, Glas, Metall oder keramischen Werkstoff vorgesehen, durch welche Licht aus dem Lichtleiter 30 in der Richtung der Längsachse des Lichtleiters 30 sowie unter bestimmten seitlichen Winkeln hindurchtreten kann, jedoch Licht abgeschattet wird, welches stirnseitig in das proximale Ende des Streukörpers eintreten kann.

Am distalen, dem proximalen Ende gegenüberliegenden Ende des Diffusor-Grundkörpers 43 ist zur Optimierung der Abstrahlcharakteristik eine Reflektorfläche 47 vorgesehen, welche gerichtet reflektierend als Spiegelelement in Form eines Metallblättchens oder als dünne Spiegelfolie beispielsweise einer Trägerfolie mit aufgedampfter Spiegelschicht oder einer Beschichtung mit einer Reflektivität > 95% ausgebildet sein kann.

Als vorteilhaft hat sich auch eine diffus reflektierende Schicht beispielsweise mittels Aufbringung zum Beispiel Bedruckung mit bevorzugt weißer Farbe, herausgestellt.

In einer weiteren Ausgestaltungsvariante kann vorgesehen sein, dass die Reflektorfläche 47 als kurze polierte Drahtabschnitte aus Aluminium oder Gold hergestellt sind, die direkt in Kontakt mit dem Diffusor-Grundkörper 43 gebracht sind. Damit ergeben sich zudem kleine Wärmesenken, die Hotspots vermeiden helfen. Weiterhin haben sich als besonders vorteilhaft aufgesputterte oder aufgedampfte dielektrische Reflektionsschichten auf dem distalen Ende des Diffusor-Grundkörpers 43 herausgestellt, welche aus mehreren Schichten bestehen und hinsichtlich der Reflektivität auf die Wellenlänge des verwendeten Lichtes abgestimmt sind. Der Begriff "hinsichtlich der Reflektivität auf die Wellenlänge des verwendeten Lichtes abgestimmt" gibt im Rahmen der vorliegenden Offenbarung an, dass beim Abstimmen eine möglichst hohe Reflektivität bei dieser Wellenlänge erreicht wird oder sogar das Maximum der Reflektivität bei der Wellenlänge liegt, auf welche jeweils abgestimmt wird. Beispiele einer solchen Reflektorschicht ist ein Mehrfach-Schichtsystem aus alternierend aufgebrachten TiO₂- und SiO₂-Schichten, welches z.B. eine Reflektivität von > 99% im Anwendungswellenlängenbereich, z.B. für rotes Licht mit (690 ± 10) nm, aufweist. Derartige Schichtsysteme können auf die jeweilige Anwendungswellenlänge entsprechend angepaßt, dies bedeutet wie vorstehend angegeben abgestimmt werden. Hiermit kann eine ideale Rückreflektion einerseits und eine Vermeidung von Hotspots andererseits erreicht werden. Alternativen dazu oder ergänzend können auch Silberschichten mit rückseitiger Passivierung als Reflektorfläche 47 vorgesehen sein.

Zum weiteren mechanischen Schutz und/ oder zur Homogenisierung der Abstrahlcharakteristik kann eine Hülle 49 aus transparenten und/ oder transluzentem, eingefärbten oder farblosen Material (Silikon, Glas oder Quarzglas) vorgesehen sein, die den Diffusor-Grundkörper zumindest teilweise oder abschnittsweise umschließt. Insbesondere kann mit einem transluzenten und/oder Streuzentren enthaltenden Material eine zusätzliche Homogenisierung erzielt werden. Geeignet sind beispielsweise entsprechende Körper oder Schläuche aus Silikon, Teflon oder auch aus einem Polyether-Block-Amid-BlockCopolymer, welches beispielsweise als PEBAX^{®} im Handel bekannt ist. Als Hülle 49 haben sich, zumindest abschnittsweise aufgebrachte, dünnwandige Schrumpfschläuchen, z.B. aus PET, erwiesen, welche einlagig oder mehrlagig ausgeführt sein können. Die Lichtauskopplung 42 gemäß eines Lambertschen Strahlers wird so weiter unterstützt beziehungsweise umgesetzt. Eine aktive Länge 40.2 des Diffusor-Elementes 40 ergibt sich dann als Abstand zwischen der Hülse 48 und der Reflektorfläche 47 und kann sich beispielsweise bis über die gesamte Länge des Diffusor-Elements 40 oder über eine aktive Länge 40.2 des Diffusor-Elements 40 erstrecken.

Zwischen Diffusor-Grundkörper 43 und der Hülle 49 aus beispielsweise Glas oder Kunststoff kann vorgesehen sein, dass zur Unterdrückung von etwaigen Oberflächenunregelmäßigkeiten, wie beispielsweise Schmutz, Rauigkeit oder ähnlichem auf dem Diffusor-Grundkörper 43, welche das Abstrahlverhalten ungünstig beeinflussen, eine Immersionsschicht zwischen Hülle 49 und Diffusor-Grundkörper 43 eingebracht ist. Dabei ist auf einen an das Glassystem angepassten Brechungsindex einerseits, auf eine hohe Transparenz sowie auf eine ausreichend hohe Viskosität im Hinblick einer guten Applizierbarkeit zu achten. Als geeignet hat sich bspw. Glyzerin oder Silikone (Öle oder Kleber) als Immersionsschicht herausgestellt.

Zur Vermeidung von Störreflexen kann zusätzlich vorgesehen sein, dass die Reflektorfläche 47 mit einer, auf einer kurzen Länge seitlich den Diffusor-Grundkörper 43 an dessen Umfangsfläche umgreifenden Hülle oder Kappe 47.2 abgedeckt ist oder von dieser gebildet wird. In diesem Fall entspricht die aktive Länge 40.2 dem Abstand zwischen der Hülse 48 und dieser Schutzkappe. Sind die Hülse 48 beziehungsweise die Kappe 47.2 aus Metall gefertigt, kann damit eine Radio-Marker-Funktion erzielt werden, was die aktive Länge 40.2 beziehungsweise die Position des Diffusor-Elementes 40 im Röntgenbild erkennbar macht. Der gesamte Durchmesser 40.1 des Diffusor-Elementes 40 beträgt für PDT-Anwendungen typischerweise zwischen 0,8 und 1,2 mm. Üblich sind Durchmesser 40.1 von knapp 1 mm. Entscheidend ist hier der Durchmesser der Kanüle, durch die die Diffusor-Elemente 40 dem Patienten appliziert werden.

Die Befestigung von Diffusor-Grundkörper 43 und Lichtleiter 30 erfolgt innerhalb der Verbindungszone 44 durch beispielsweise einen Spleiß- oder Verklebe-Prozeß mit einem brechwertangepaßten hochtransparenten Kleber. Beim Spleißen wird mittels einer Corona-Entladung und/ oder mittels eines Lasers, üblicherweise mit einem CO₂-Laser, der Lichtleiter 30 und der Diffusor-Grundkörper 43 an- beziehungsweise aufgeschmolzen und zusammengeführt. Je nach Material welches für den Diffusor-Grundkörper 43 und dem Lichtleiter 30 verwendet wird, kann es erforderlich sein, dass zur Anpassung derer Wärmeausdehnungskoeffizienten ein Zwischenmedium 45 verwendet wird. Dieses kann beispielsweise bei einer Glas-/ Quarz-Verschmelzung ein Lot- oder Übergangsglas oder ein optischer Kleber oder Kitt sein. Denkbar und vorteilhaft zu verwirklichen ist auch eine mechanische Verpressung in Form einer Muffe, wobei lediglich der Übergang mit einem optischen Kit zur Vermeidung von Reflektionsverlusten gefüllt ist. Ebenso kann ein in der Verbindungszone 44 zwischen proximalem Ende des Diffusor-Grundkörper 46 und dem distalen Ende des Lichtleiters 30 angeordnetes optisches Element einge- beziehungsweise verbunden werden

In den Figuren 3a bis 3d sind verschiedene Ausführungsbeispiele für die Anordnung zumindest eines Streuelementes 43.6 in einem Diffusor-Grundkörper 43 dargestellt. Der Diffusor-Grundkörper 43 weist jeweils ein Hüllrohr 43.3 und eine Matrix 43.4 auf, in die die Streuelemente 43.6 in bevorzugter Anordnung parallel zur Längsachse 43.2 (siehe Fig. 2) über die gesamte Länge des Diffusor-Grundkörpers 43 eingebettet sind. Die axiale Ausdehnung einzelner Streuelemente kann dabei kleiner als die gesamte Länge des Diffusor-Grundkörpers 43 sein.

Fig. 3a zeigt eine Anordnung, bei der über den Durchmesser 43.1 des Diffusor-Grundkörpers 43 eine Mehrzahl Streuelemente 43.6 mehr oder weniger statistisch gleichmäßig verteilt angeordnet sind, das heißt dass eine Mehrzahl von Streuelementen 43.6 vorhanden ist, die um die Längsachse angeordnet sind; bevorzugt sind die Streuelemente in einer regelmäßigen Struktur um die Längsachse angeordnet.

Fig. 3b zeigt eine Anordnung, bei der einzelne Streuelemente 43.6 eine insbesondere ringförmige Anordnung ausbilden, das heißt dass eine Mehrzahl von Streuelementen vorhanden ist, die um die Längsachse, bevorzugt kreisförmig angeordnet sind.

Alternativ zu Fig. 3b zeigt Fig. 3c eine Anordnung bei der nur ein Streuelement 43.6 in Form eines Rohrabschnittes in die Matrix 43.4 eingebettet ist, das heißt dass das zumindest eine Streuelement (43.6) rohrförmig um und insbesondere koaxial zur Längsachse angeordnet ist. Vorteilhaft bei dieser Anordnung ist eine besonders kostengünstige und reproduzierbare Herstellung der Vorform des Diffusor-Grundkörpers 43, da hier der Herstellprozess erheblich vereinfacht werden kann.

Grundsätzlich sind auch andere Geometrien für das zumindest eine Streuelement 43.6 und/oder die Anordnung einer Mehrzahl von Streuelementen 43.6, wie beispielsweise sechseckig, quadratisch, dreieckig denkbar und vorteilhaft realisierbar.

Fig. 3d zeigt eine Anordnung, bei der die Streuzentren 43.6 mehr oder weniger gleichverteilt in der Matrix 43.4 angeordnet sind, dabei aber eine Kernzone 43.7 um die Längsachse 43.2 des Diffusor-Grundkörpers 43 freilassen, das heißt dass die Anzahl der Streuelemente 43.6 je Flächeneinheit der Querschnittsfläche des Diffusor-Grundkörpers 43 außerhalb einer Kernzone 43.7 entlang der Längsachse grösser als die je Flächeneinheit in der Kernzone 43.7 ist.

Diese Anordnung hat den Vorteil, dass das Laser-Licht, welches üblicherweise nur eine kleine numerische Apertur (NA, typ. < 0,3) aufweist, zunächst nach der Einkopplung in den Diffusor-Grundkörper 43 wenig an den Streuelementen 43.6 im Außenbereich um die Kernzone 43.7 gestreut wird, und erst nach einiger Entfernung von der Einkoppelfläche 46 (siehe Fig. 2) verstärkt gestreut wird, wenn die einzelnen Strahlen die Streuelemente 43.6 im Randbereich erreichen. Damit kann eine Intensitätsabsenkung des seitlich abgestrahlten Lichtes direkt nach der Einkoppelfläche 46 und damit eine Homogenisierung des Intensitätsverlaufs entlang des Diffusors erreicht werden.

Bei einer konstanten Konzentration von Streuelementen entlang der Längsachse des Diffusor-Grundkörpers weist der Intensitätsverlauf einen typischerweise exponentiellen Abfall mit l₍ₗ₎ = l₀ x e ^{-l/k} auf. Dabei hat sich als günstiger Wert für k herausgestellt, wenn k in etwa der Länge des Diffusor-Grundkörpers (im konkreten Beispiel 40 mm) entspricht. Daraus ergibt sich in etwa ein 1/e-Abfall der im Betriebszustand seitlich abgestrahlten Intensität der Strahlung entlang des Diffusor-Grundkörpers, was durch die weiteren Maßnahmen derart korrigiert werden kann, dass die o.g. Homogenitätsanforderungen insbesondere für PDT-Anwendungen erfüllt werden können. In einem bevorzugten Ausführungsbeispiel wurde mit 21 Streuelementen mit jeweils einen Durchmesser von 0,3 mm als Ausgangsmaterial für die Vorform und einem Matrix-Durchmesser von etwa 600 µm (Ausgangsgeometrie Vorform 34 mm Durchmesser) ein k-Wert von 42 mm ermittelt.

Fig. 4a und 4b zeigen schematisch 2 Ausführungsbeispiele für den Aufbau der Matrix 43.4 im Diffusor-Grundkörper 43 in einem Querschnitt senkrecht zu der Längsachse des Diffusor-Grundkörpers 43.

Fig. 4a zeigt beispielhaft ein Streuelement 43.6 welches in der Vorform als dünnes Stäbchen zwischen den Matrix-Elementen 43.5 in Form von Einzelstäben eingelagert ist. Im gezeigten Beispiel füllt das Streuelement 43.6 die Zwischenräume (Zwickel) von drei Einzelstäben als Matrix-Elemente 43.5 aus. Im gezeigten Beispiel wurden für die Herstellung der Vorform Einzelglasstäbe von 2 mm Durchmesser als Matrix-Elemente 43.5 verwendet. Die Streuelemente sind aus 0,3 mm dicken Weißglasstäben gebildet. Nach dem thermischen Ziehprozess, d.h. nach dem Herunterziehen auf den Durchmesser 43.1 des Diffusor-Grundkörpers 43 ist das Streuelement 43.6 an- oder aufgeschmolzen und weist einen dreieckigen, beispielsweise insbesondere hyperbolisch dreieckigen Querschnitt auf.

Eine Ausführungsform der der als Weißglasstäbe ausgebildeten Streuelemente 43.6 oder des Weißglasrohrs sieht vor, dass in diesen Streuzentren durch Streupartikel gebildet werden, wobei die Konzentrationen der Streupartikel im Streubereich von 10 ppm bis 1000 ppm und bevorzugt von 20 ppm bis 100 ppm beträgt.

Die Effizienz der Auskopplung aus dem Streubereich, somit dem Volumen des Weißglases der Streustäbe oder des Weißglasrohrs ist neben der streuenden Eigenschaft der Streupartikel als intrinsischem Parameter auch von der Konzentration der Streupartikel im Streubereich selbst abhängig.

Die Konzentrationsangabe in ppm bezieht sich hierbei auf den Anteil der Streupartikel im Verhältnis zu den Masseanteilen der Bestandteile des Weißglases, in welchem die Streupartikel eingelagert sind.

Dienen inhomogene Bereiche des Weißglases als Streuzentren, ergibt sich eine alternative Ausführungsform, in der die inhomogenen Bereiche bevorzugt durch Phasentrennung und/oder Entmischung der Glaskomponenten des Glases gebildet werden, in welches sie eingelagert sind.

Die durch inhomogene Bereiche gebildeten Streuzentren weisen bevorzugt einen Durchmesser von 10 nm bis 1000 nm auf, besonders bevorzugt von 100 nm bis 800 nm.

Besonders bevorzugt sind diese Streuzentren kugelförmig. Für nicht kugelförmige Streuzentren wird als Durchmesser ihre maximale Ausdehnung verstanden.

Das Glas, welche vorliegend als Weißglas bezeichnet wird, in dem die inhomogenen Bereiche als Streuzentren eingelagert sind, kann bevozugt aus einem As- und Pb-haltigen Silikatglas bestehen. Die Streuzentren weisen in diesem Fall bevorzugt einen gegenüber der umgebenden Glasmatrix erhöhten Gehalt an Pb und/oder As auf.

Alternativ kann das Glas beziehungsweise Weißglas, in welches die inhomogenen Bereiche als Streuzentren eingelagert sind, aus einem Fluorhaltigen Ca-Zn-Silikatglas bestehen. Dann weisen die Streuzentren einen gegenüber der umgebenden Glasmatrix bevorzugt einen erhöhten Gehalt an Fluor auf.

Fig. 4b zeigt eine alternative Anordnung, bei der die Durchmesser der Streuelemente 43.6 gleich groß oder kleiner sind als die Durchmesser der als Einzelstäbe ausgebildeten Matrix-Elemente 43.5. Hier liegen die typischen Durchmesser vor dem Ziehprozess in der entsprechend zusammengesetzten Vorform im Bereich von 0,5 bis 1 mm für die Streuelemente 43.6 als beispielsweise Weissglasstäbchen und die Matrix-Elemente 43.5. Nach dem thermischen Ziehprozess, das heißt nach dem Herunterziehen auf den Durchmesser 43.1 des Diffusor-Grundkörpers 43 ist das Streuelement 43.6 an- oder aufgeschmolzen und weist einen sechseckigen, beispielsweise insbesondere hyperbolisch sechseckigen Querschnitt auf.

Die Anordnung der Streustäbe in den Zwickeln der Vorform ermöglicht dabei bei gegebener Größe der Lichtleitstäbe und gegebenem Querschnittsanteil eine höhere Anzahl der Streukörper und damit eine bessere Homogenität zu erzielen. Die Matrix-Elemente 43.5 und die Streuelemente 43.6 können nach dem Ziehprozess als Diffusor-Grundkörper 43 einen runden, sechseckigen, quadratischen oder dreieckigen Querschnitt, insbesondere dessen hyperbolische Varianten, aufweisen.

Zur Homogenisierung des Intensitätsverlaufs kann, wie dies die Figuren 5a und 5b zeigen, vorgesehen sein, dass die Reflektionsfläche 47 konkav (Fig. 5a) oder konvex (Fig. 5b) ausgeformt ausgebildet sind. Damit kann erreicht werden, dass reflektierte Strahlen mit nahezu parallelem Verlauf zur Längsachse 43.2 unter steilerem Winkel zur Längsachse 43.2 zurückreflektiert und damit an den Streuelementen 43.6 häufiger gestreut werden, so dass die Auskoppeleffizienz am distalen Ende des Diffusor-Elementes 40 erhöht wird.

Die Reflektorfläche 47 am distalen Ende des Diffusor-Grundkörpers 43 kann auch als hohler und/ oder transparenter Körper 47.1 mit einer in den Hohlraum und/ oder in den transparenten Körper hinein reflektierenden Hülle 47.2 ausgebildet sein, wie dies Fig. 5c schematisch zeigt. Die Hülle 47.2 kann als bevorzugt gerichtet oder diffus reflektierende Beschichtung und/ oder Kappe ausgeführt sein. Diese können auch ohne Hohlraum direkt mit dem Diffusor-Grundkörpers 43 abschließen und diesen in beiden Fällen über eine kurze Länge am distalen Enden zumindest teil- oder abschnittsweise an dessen Umfang radial umfassen.

Damit liegt die Reflektorfläche 47 konkav oder konvex und/ oder als direkt oder beabstandet, einen Hohlraum zwischen Reflektorfläche 47 und distalem Ende des Diffusor-Grundkörpers 43 bildend, an den Diffusor-Grundkörper 43 anschließender, Körper 47.1 und/ oder Hülle 47.2 als einseitig geschlossener Hohlkörper ausgebildet vor.

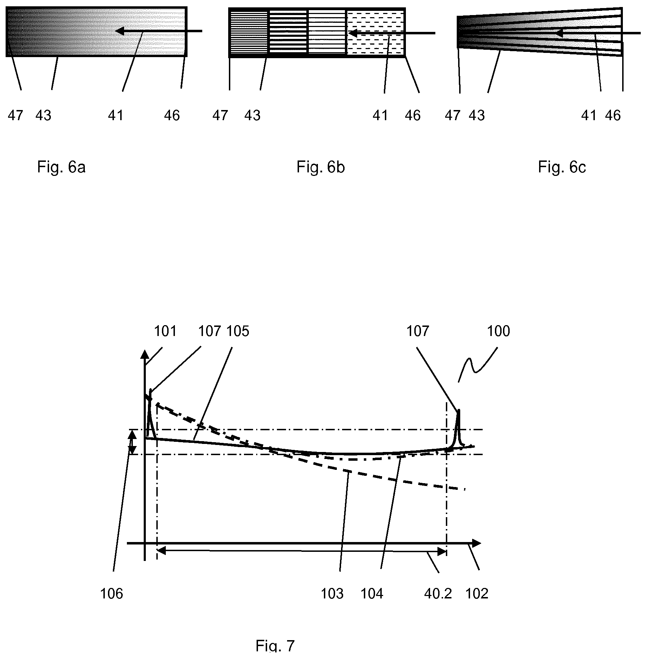

Um eine weitere Homogenisierung im Hinblick auf einen möglichst konstanten Intensitätsverlaufs entlang des Diffusors zu erzielen, können weitere Prozessschritte angewendet werden, wie dies in den Figuren 6a bis 6c schematisch dargestellt ist.

So kann beispielsweise je nach dem eingesetzten Material und den Materialeigenschaften der Streuelemente 43.6 und der dieser umgebenden Matrix 43.4 mittels einer Gradiententemperung erreicht werden, dass durch unterschiedliche Temperaturbeaufschlagung über die Länge des Diffusors die Streuwirkung beispielsweise stetig variiert werden kann. Damit ist es möglich, dass zum Beispiel direkt nach der Einkoppelfläche 46 eine zunächst eher niedrige Streuwirkung und am anderen Ende des Diffusor-Grundkörpers 43, das heißt an der Reflektorfläche 47 eine eher höhere Streuwirkung einstellbar ist. Fig. 6a zeigt diese Möglichkeit schematisch. Damit können Diffusor-Grundkörper erhalten werden die Streuelemente mit Streuzentren mit einer Streuzentrendichte pro Volumeneinheit aufweisen, wobei sich die Streuzentrendichte am proximalen Ende des Diffusor-Grundkörpers von derjenigen am distalen Ende unterscheidet; bevorzugt ist die Streuzentrendichte am distalen Ende grösser als am proximalen Enden; besonders bevorzugt liegt ein Gradient der Streuzentrendichte vor.

Fig. 6b zeigt einen "Kaskaden"-Ansatz, bei dem unterschiedlich hergestellte Diffusor-Abschnitte mit jeweils unterschiedlicher Anordnung und/oder Dichte der Streuelemente 43.6 in der Matrix 43.4 mittels Spleißen oder Verkleben mit einem brechwertangepassten Kleber abschnittsweise zu einem Diffusor-Grundkörper 43 zusammengesetzt sind, wobei die Streuwirkung des ersten Diffusor-Abschnitts, in dem die Lichteinkopplung 41 erfolgt, am geringsten ausgebildet sein kann. In Richtung der Lichtausbreitung im Diffusor-Grundkörper 43 nimmt bei entsprechende Auswahl der Abschnitte dann in den einzelnen Diffusor-Abschnitten die Streuwirkung zu. Damit kann in Stufen die Abstrahlintensität innerhalb eines Toleranzbandes die Homogenität des axial und radial abgestrahlten Lichtes im Betriebszustand konstant gehalten werden. Hiermit kann ein Diffusor-Grundkörper 43 aus einer Mehrzahl von Abschnitten aus sich unterscheidenden Diffusor-Grundkörpern gebildet werden.

Fig. 6c zeigt eine weitere Möglichkeit, einen möglichst konstanten Intensitätsverlauf entlang des Diffusors zu erzielen. Dabei werden die aus der Vorform ausgezogenen Diffusor-Stäbe durch Variation der Ziehparameter Geschwindigkeit, Temperatur und/ oder Kraft abschnittsweise verjüngt und danach zugeschnitten und an den Enden bearbeitet. Man erhält kegelförmig verjüngte Diffusor-Grundkörper 43, die aufgrund der Verjüngung eine Winkelaufweitung der Lichtstrahlen bewirken, so dass das Licht eine größere Wegstrecke zurücklegt und es daher in Längsrichtung des Diffusor-Grundkörpers 43 zu einer stärkeren Streuung kommt. Die Streuelemente verlaufen dann weiterhin gleichmäßig angeordnet, idealerweise, auf einen gemeinsamen Fluchtpunkt hin. Somit kann ein Diffusor-Grundkörper 43 zumindest teilweise oder abschnittsweise kegelförmig ausgebildet werden, in welchem das zumindest eine Streuelement 43.6 entlang der Längsachse 43.2 des Diffusor-Grundkörpers 43 im Wesentlichen in einem Winkel zur Längsachse angeordnet ist.

Der üblicherweise zu erwartende exponentielle Intensitätsabfall vom proximalen zum distalen Ende des Diffusor-Grundkörpers 43 kann mit obigen Beispielen und/ oder Kombination daraus zumindest teilweise kompensiert werden.

Darüber hinaus sind weitere nachgeschaltete Prozesse, wie bereits oben beschrieben, denkbar und vorteilhaft durchführbar, bei denen der Intensitätsverlauf der seitlichen Abstrahlung des Diffusor-Grundkörper 43 und/ oder des Diffusor-Elements 40, durch Strukturierung in deren Volumen und/ oder an deren Oberflächen, korrigiert beziehungsweise angepasst werden kann.

In Fig. 7 sind schematisch in einem Verlaufsdiagramm 100 unterschiedliche Verläufe 103, 104, 105 der Intensität 101 der im Betriebszustand seitlich abgestrahlten Strahlung in Abhängigkeit vom Abstand zur Einkoppelfläche 102 dargestellt.

Ein erster Verlauf 103 zeigt einen typischerweise exponentiellen Abfall der Intensität 101, wie er sich als Lösung einer Differentialgleichung für einen über die Länge homogenen Streuverlauf, d.h. ein konstantes Verhältnis von eingestrahlter Strahlung zu gestreuter Strahlung in einem Längenabschnitt liegt vor, ergibt.

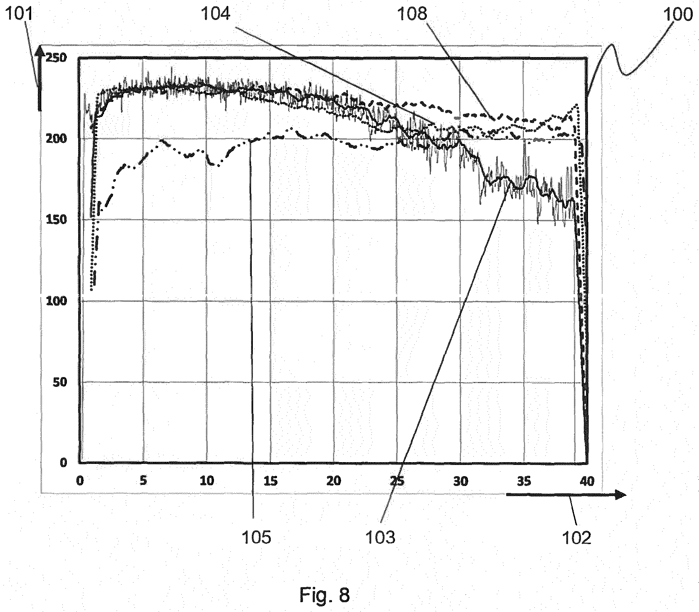

Durch Anbringen einer Reflektorfläche 47 am distalen Ende des Diffusor-Grundkörpers 43 (vergl. Fig. 2) kann ein Teil der Strahlung wieder zurückreflektiert werden, welche dann insbesondere im Bereich vor der Reflektorfläche 47 zusätzliche Streubeiträge liefert. Mathematisch bedeutet dies eine Addition von zwei Exponential-Funktionen, was einem zweiten Verlauf 104 entspricht, wie dieser auch in Figur 8 dargestellt ist..

In einem dritten Verlauf 105, welcher in Figur 8 dargestellt ist, ist ein Intensitätsverlauf für eine weiter optimierte Ausführung des Diffusor-Grundkörpers 43 gezeigt. Durch geometrische Anordnungen der Streuelemente 43.6, wie sie insbesondere in Fig. 3d beschrieben sind, kann die Verlaufskurve der Intensität 101 nahe der Einkoppelfläche 46, das heißt am proximalen Ende des Diffusor-Grundkörpers 43, abgeflacht oder sogar zunächst ansteigend im oder in den Intensitätstoleranzbereich 106 eingestellt werden, so dass insgesamt im Wesentlichen über die aktive Länge 40.2 des Diffusor-Elementes 40 eine vergleichsweise kleine Intensitätsschwankung beziehungsweise gute Homogenität der seitlichen Abstrahlung des eingekoppelten Lichtes innerhalb des Intensitätstoleranzbereich 106 erzielt werden kann.

Trotzdem können insbesondere bei oder nahe der Einkoppelfläche 46 und auch an oder nahe der Reflektorfläche 47 Intensitätsspitzen 107 auftreten, die beispielsweise durch konstruktive Maßnahmen, beispielsweise Hülsen 48 oder Kappen beziehungsweise Hüllen 47.2, wie sie in und bezüglich Fig. 2 und Fig. 5c beschrieben sind, abgeschirmt beziehungsweise minimiert werden können.

Fig. 8 zeigt in einem weiteren Verlaufsdiagramm 100 vier gemessene Verläufe der Intensität 101 der im Betriebszustand seitlich abgestrahlten Strahlung in Abhängigkeit vom Abstand zur Einkoppelfläche 102. Die Einkopplung erfolgte je mit einem Lichtleiter 30 mit ca. 360 µm Kerndurchmessern 31.1 in den Diffusor-Grundkörper 43 mit ca. 600 µm Durchmesser. Die Intensität 101 ist hier als Grauwertintensität beispielsweise einer CCD-Kamera ermittelt und dargestellt.

Es wurde konkret bei den Messungen der Fig. 8 jeweils mit monochromatischem Licht mit einer Wellenlänge von 685 nm gemessen. Als Kamera wurde eine Kamera der Firma Nikon vom Typ 1V1 und jeweils nur der von dieser Kamera ausgegebene Rot-Kanal verwendet.

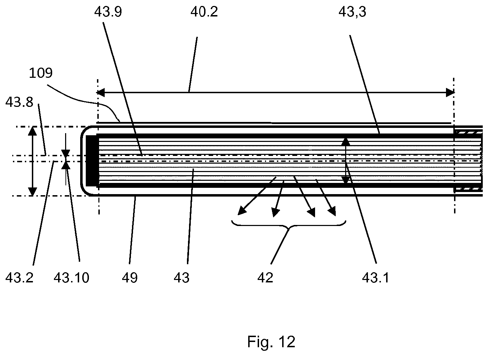

Es wurde dabei jeweils eine sich parallel zur Längsachse 43.2 des Diffusor-Grundkörpers 43 erstreckende und in Fig. 12 dargestellte Gerade 109, die am Ort des Lichtaustritts, insbesondere des gestreuten Lichtes lag, vermessen. Hierbei ergab sich durch das abbildende optische System bei dieser Messung eine in Richtung dieser Geraden 109 verlaufende örtliche Auflösung der Messungen von 400 Pixeln pro cm. Für das abbildende optische System wurde ein Objektiv mit einer Brennweite von 30 mm eingesetzt, welches jeweils mit einer Blendenöffnung von 5,6 betrieben wurde.

Die vorliegend offenbarte und in den Ansprüchen erwähnte Intensität oder Intensitätsverteilung entspricht in physikalischer Hinsicht auch der Leuchtdichte, welche auch als Luminanz oder der Helligkeit bezeichnet wird, soweit wie vorliegend mit einem optischen System gemessen wird, welches einen feststehenden Raumwinkel erfasst, welches durch die Verwendung der Blendenöffnung von 5,6 bei feststehender Brennweite von 30 mm verwirklicht wurde.

Als Verteilung der Leuchtdichte ergibt sich daraus jeweils die in Fig. 8 dargestellte Verteilung entlang der für diese Messung verwendeten Geraden 109.

Da für die vorliegende Offenbarung jedoch die relativen Werte zwischen der mittleren seitlichen Abstrahlungsintensität des Beleuchtungssystems und eine prozentuale Abweichung von dieser mittleren seitlichen Abstrahlungsintensität angegeben werden, wird für diese Angabe jeweils derselbe prozentuale Abweichungswert erhalten, wenn hierbei die Intensität, Leuchtdichte, Luminanz oder Helligkeit eines Punktes auf der gemessenen Geraden 109 gemessen wird.

Als mittlerer seitliche Abstrahlungsintensität wird der Mittelwert aller auf der Geraden 109 gemessen Werte zugrunde gelegt. Bei der Bestimmung dieses Mittelwerts wurden jedoch die örtlich am Anfang und am Ende der Messtrecke liegenden Messpunkte, bei welchen ein starker Abfall der Intensität zu beobachten ist, nicht mit erfasst. Hierbei wurden jeweils bei einer ca. 40 mm langen Messtrecke auf der Geraden 109 die Werte der ersten und der letzten 2 mm nicht in die Mittelung mit einbezogen.

Die Angabe, dass das Beleuchtungssystem im Betriebszustand eine Intensitätsverteilung der seitlichen Abstrahlung aufweist, die um höchstens ± 50%, bevorzugt höchstens ± 30% und meist bevorzugt höchstens als ± 5% von abweicht, legt für diese Abweichung einen gleitenden Durchschnitt zugrunde, wie dieser nachfolgend noch detaillierter beschrieben wird.

Als gleitender Durchschnitt wird hierbei die Mittelung über zehn, auf der Geraden 109 seitlich neben einander liegender gemessener Bildpunkte verstanden.

Da es sich bei dieser Mittelung um eine arithmetische Mittelung handelt, können hierbei für jeweils einen Bildpunkt zehn nebeneinander liegende Bildpunkte gemittelt werden und für den nächsten seitlich benachbarten gemessenen Bildpunkt diese zehn Bildpunkte um einen Bildpunkt seitlich verschoben und ebenfalls arithmetisch gemittelt werden.

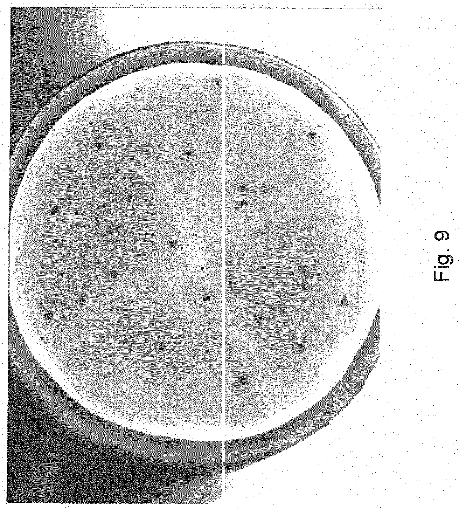

Der Diffusor-Grundkörper 43 hat im gezeigten Beispiel eine Länge von circa 40 mm. 21 Streuelemente 43.6 sind in einer Anordnung gemäß Fig. 3d im Diffusor-Grundkörper 43 angeordnet, wobei die Streuelemente 43.6 als 0,3 mm dicke Weißglasstäbchen und die Matrix 43.5 als 2 mm dicke Lichtleitstäbe in der Vorform konfiguriert wurden, analog der in Fig. 4a gezeigten Anordnung. Zur besseren Übersichtlichkeit sind die Kurven als gleitender Durchschnitt dargestellt, wobei dem Verlauf 103 beispielhaft die Rohdaten unterlegt sind. Der Verlauf 103 zeigt den Intensitätsverlauf ohne Reflektorfläche 47 bei einer Anordnung der Streuelemente gemäß Fig. 3a und Fig. 9.

Der Verlauf 104 zeigt den Intensitätsverlauf eines Diffusor-Grundkörpers nach Fig. 3a bzw. Fig. 9 mit Reflektorfläche 47, welche als aufgeklebte Spiegelfolie ausgebildet ist. Blendet man die steil ansteigenden Anfangsbereiche bzw. abfallenden Endbereiche und damit auch gegebenenfalls vorliegende Intensitätsspitzen aus, so ergibt sich hier ohne weitere Zusatzmaßnahmen eine Intensitätsschwankung von kleiner ± 20 % vom Mittelwert.

Der Verlauf 108 zeigt den Intensitätsverlauf eines Diffusor-Grundkörpers nach Fig. 3a beziehungsweise Fig. 9 mit Reflektorfläche 47, welche als diffus reflektierende, weiße Beschichtung ausgebildet ist. Blendet man die steil ansteigenden Anfangsbereiche beziehungsweise abfallenden Endbereiche und damit auch gegebenenfalls vorliegende Intensitätsspitzen aus, so ergibt sich hier ohne weitere Zusatzmaßnahmen eine Intensitätsschwankung von kleiner ± 10 % vom Mittelwert.

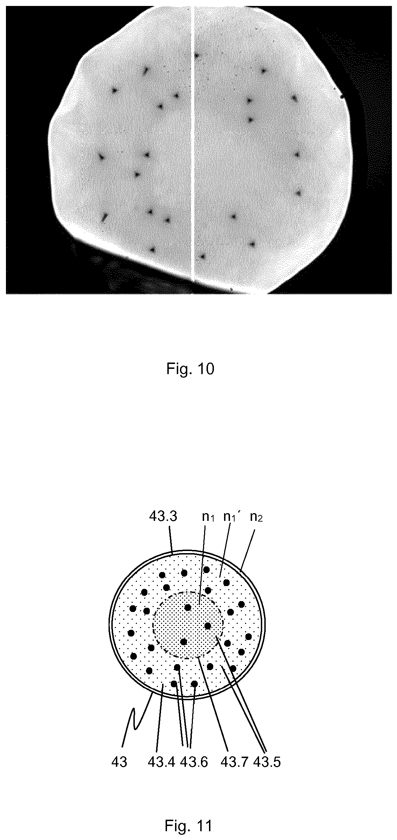

Der Verlauf 105 zeigt den Intensitätsverlauf eines Diffusor-Grundkörpers nach Fig. 3d beziehungsweise Fig. 10 mit Reflektorfläche 47, welche als diffus reflektierende, weiße Beschichtung ausgebildet ist. Des Weiteren wird die Wirkung der Anordnung nach Fig. 3d deutlich.

Fig. 11 zeigt schematisch einen Querschnitt durch einen Diffusor-Grundkörper 43, bei dem der Diffusor-Grundkörper 43 bezogen auf seine Querschnittsfläche eine Matrix 43.4 aufweist, welche zwischen der Kernzone 43.7 und dem Randbereich der Matrix unterschiedliche Brechungsindices n₁ und n₁' aufweist, in die die Streuelemente 43.6 eingelagert sind. Damit kann gezielt die numerische Apertur NA in der Kernzone 43.7 mit einem Matrix-Brechungsindex n₁ und in der Randzone der Matrix mit einem Brechungsindex n₁' beeinflusst werden und somit gezielt die Ausbreitung des Lichtes im Diffusor-Grundkörper 43 und damit eine Anregung der Streuzentren 43.6 über die Länge des Diffusor-Grundkörpers 43.3 an die erforderliche Abstrahlcharakteristik angepasst werden. Zudem kann beim Herstellprozeß auch eine beliebige Querschnittsgeometrie der Kernzone 43.7 dem Brechungsindex n₁, das heißt von im Wesentlichen kreisrunden, wie in Fig. 11 gezeigt, bis hin zu einer polygonen oder sternförmigen Form realisiert werden. Beispielsweise lassen sich aus Matrix-Elementen 43.5 in der Kernzone 43.7, welche aus Glasstäben mit einem Brechungsindex n₁ = 1,625 gebildet sind, und aus Matrix-Elementen 43.5 in der Randzone, welche aus Glasstäben mit einem Brechungsindex n₁' = 1,588 unterschiedliche numerische Aperturen in der Kernzone 43.5 und in der Randzone realisieren, wobei in diesem Beispiel der Brechungsindex n₂ des Hüllrohrs 43.3 gleich 1,49 beträgt. Im beschrieben Beispiel beträgt die NA der Kernzone 43.7 0,35 und die der Randzone 0,55. Damit können gezielt die Lichtausbreitung und damit die Anregung der Streuzentren 43.3 beeinflusst werden.

Generell ist vorzugsweise das zumindest eine Streuelement 43.6, zumindest wenn dieses als Weißglasstäbchen oder Weißglasrohr ausgebildet ist, entlang der Längsachse 43.2 des Diffusor-Grundkörpers 43 im Wesentlichen zu dieser parallel ausgerichtet. Dies bedeutet, dass wie beispielsweise in Figur 12 dargestellt die Längsachse 43.8 eines Weißglasstäbchens 43.9 zur Längsachse 43.2 des Diffusor-Grundkörpers 43 einen Winkel 43.10 einschließt, welcher kleiner ist als 1°.

Wenn das zumindest eine Streuelement 43.6, zumindest wenn dieses als Weißglasstäbchen oder Weißglasrohr ausgebildet ist, entlang der Längsachse 43.2 des Diffusor-Grundkörpers 43 in einem Winkel 40.10, zur Längsachse des Diffusor-Grundkörpers 43 angeordnet, bedeutet dies, dass wie beispielsweise in Figur 12 dargestellt die Längsachse 43.8 eines Weißglasstäbchens 43.9 zur Längsachse 43.2 des Diffusor-Grundkörpers 43 einen Winkel 43.10 einschließt, welcher kleiner ist als 10°.

Gleiches gilt auch für die in den Figuren nicht dargestellte Längsachse eines Weißglasrohrs, wenn dieses ein Streuelement 43.6 ausbildet.

Das erfindungsgemäße Beleuchtungssystem hat den Vorteil, dass die DiffusorElemente 40 mit den Diffusor-Grundkörpern 43 zum einen kostengünstig und reproduzierbar herstellbar sind und andererseits hinsichtlich Abstrahlcharakteristik der Intensität der im Betriebszustand seitlichen Abstrahlung homogen ausgebildet werden können. Das Beleuchtungssystem kann im Betriebszustand eine Intensitätsverteilung der seitlichen Abstrahlung aufweisen, die um höchstens ± 50%, bevorzugt höchstens ± 30% und meist bevorzugt höchstens ± 5% von der mittleren seitlichen Abstrahlungsintensität abweicht. Damit können insbesondere Anwendungen im PDT-Bereich adressiert werden. Aber auch Anwendungen mit höheren Laser-Leistungen, zum Beispiel beim EVLT, sind mit diesen Diffusor-Elementen 40 möglich.

### Bezugszeichenliste:

| | |
|---|---|
| 1 | Beleuchtungssystem |
| 10 | Laser-Lichtquelle |
| 20 | Stecker |
| 30 | Lichtleiter |
| 31 | Kern |
| 31.1 | Kerndurchmesser oder Faserbündeldurchmesser |
| 32 | Mantel |
| 40 | Diffusor-Element |
| 40.1 | Durchmesser |
| 40.2 | aktive Länge |
| 41 | Lichteinkopplung |
| 42 | Lichtauskopplung |
| 43 | Diffusor-Grundkörper |
| 43.1 | Durchmesser |
| 43.2 | Längsachse |
| 43.3 | Hüllrohr |
| 43.4 | Matrix |
| 43.5 | Matrix-Element |
| 43.6 | Streuelement |
| 43.7 | Kernzone |
| 43.8 | Längsachse des Streuelements, insbesondere Weißglasstäbchens |
| 43.9 | Weißglasstäbchen |
| 43.10 | Winkel |
| 44 | Verbindungszone |
| 45 | Zwischenmedium |
| 46 | Einkoppelfläche |
| 47 | Reflektorfläche |
| 47.1 | Körper |
| 47.2 | Reflektierende Hülle |
| 48 | Hülse |
| 49 | Hülle |
| 50 | Gewebe |
| 60 | Tumorgewebe |
| 100 | Verlaufsdiagramm |
| 101 | Intensität |
| 102 | Abstand zur Einkoppelfläche |
| 103 | 1. Verlauf |
| 104 | 2. Verlauf |
| 105 | 3. Verlauf |
| 106 | Intensitätstoleranz |
| 107 | Intensitätsspitzen |
| 108 | 4. Verlauf |
| 109 | Gerade |

## Patentansprüche

1. Beleuchtungssystem (1) insbesondere für ein medizintechnisches Therapie- und/oder Diagnosesystem, umfassend wenigstens eine Laser-Lichtquelle (10), und einen Lichtleiter (30), der an einem proximalen Ende an die wenigstens eine Laser-Lichtquelle (10) anschließbar ist, und welches am distalen Ende des Lichtleiters (30) ein Diffusor-Element (40) mit einer Längsachse aufweist, die senkrecht zu einer Einkoppelfläche des Lichtleiters in dem Diffusor-Element (40) verläuft, wobei
das Diffusor-Element (40) im Betriebszustand Licht über seine aktive Länge (40.2) seitlich zur Längsachse abstrahlt, wobei
das Diffusor-Element (40) mindestens einen Diffusor-Grundkörper (43) aufweist und der Diffusor-Grundkörper (43) mindestens ein Streuelement (43.6) beinhaltet,
wobei
das zumindest eine Streuelement (43.6) entlang der kompletten Längsachse (43.2) des Diffusor-Grundkörpers (43) mit einheitlichem Querschnitt im Wesentlichen zu dieser parallel ausgerichtet ist oder bei sich verjüngenden Diffusor-Grundkörpern in einem Winkel zur Längsachse angeordnet ist,
wobei
am distalen Ende des Diffusor-Grundkörpers (43) und/ oder dem Übergangsbereich zwischen Lichtleiter (30) und Diffusor-Grundkörper (43) und/ oder den Diffusor-Grundkörper (43) zumindest teilweise oder abschnittsweise umschließend Einrichtungen zur Homogenisierung der Abstrahlungsintensität entlang der Längsachse (43.2) des Diffusor-Grundkörpers (43) vorgesehen sind, umfassend Hülsen, Hüllen, Kappen und/ oder Schichten, wobei der Diffusor-Grundkörper (43) eine Matrix (43.4) aus Glas, Quarzglas oder transparenter Glaskeramik umfasst oder aus dieser besteht, und
wobei das Beleuchtungssystem im Betriebszustand eine Intensitätsverteilung der seitlichen Abstrahlung aufweist, die um höchstens ± 50%, bevorzugt höchstens ± 30% und meist bevorzugt höchstens als ± 5% von der mittleren seitlichen Abstrahlungsintensität abweicht.

2. Beleuchtungssystem (1) nach Anspruch 1, wobei
eine Mehrzahl von Streuelementen (43.6) vorhanden ist, die um die Längsachse des Diffusor-Elements (40) angeordnet sind; bevorzugt sind die Streuelemente (43.6) in einer regelmäßigen Struktur um die Längsachse (43.2) angeordnet, insbesondere bevorzugt kreisförmig.

3. Beleuchtungssystem (1) nach Anspruch 2, wobei
die Anzahl der Streuelemente (43.6) je Flächeneinheit bezogen auf die Querschnittsfläche des Diffusor-Grundkörpers (43) außerhalb einer Kernzone (43.7) entlang der Längsachse (43.2) grösser als in der Kernzone (43.7) ist.

4. Beleuchtungssystem (1) nach Anspruch 1, wobei
das zumindest eine Streuelement (43.6) rohrförmig und insbesondere koaxial zur Längsachse (43.2) des Diffusor-Grundkörpers (43) angeordnet ist.

5. Beleuchtungssystem (1) nach mindestens einem der vorherigen Ansprüche, wobei
das zumindest eine Streuelement (43.6) einen dreieckigen, insbesondere hyperbolisch dreieckigen oder sechseckigen, insbesondere hyperbolisch sechseckigen Querschnitt aufweist.

6. Beleuchtungssystem (1) nach mindestens einem der vorherigen Ansprüche, wobei
der Diffusor-Grundkörper (43) bezogen auf seine Querschnittsfläche eine Matrix (43.4) aufweist, welche unterschiedliche Brechungsindices n₁ und n₁', insbesondere zwischen der Kernzone (43.7) und dem Randbereich der Matrix (43.4), aufweist, in die das zumindest eine Streuelement (43.6) eingelagert ist.

7. Beleuchtungssystem (1) nach den Ansprüchen 1 bis 6, wobei
der Lichtleiter (30) eine Einzelfaser mit einem Kern (31) mit einem Kerndurchmesser (31.1) und einem Mantel (32) umfaßt, wobei der Durchmesser (43.1) des Diffusor-Grundkörpers (43) im Bereich der Einkoppelfläche größer ist als oder gleich groß ist wie der Kerndurchmesser (31.1) des Lichtleiters (30) im Bereich der Einkoppelfläche (46), wobei bevorzugt das Verhältnis von Kerndurchmesser (31.1) des Lichtleiters und Durchmesser des Diffusor-Grundkörpers (43) ≤ 1,0 bis 0,7, besonders bevorzugt von ≤ 1,0 bis 0,8 beträgt, oder
der Lichtleiter ein Faserbündel (31) mit einem Faserbündeldurchmesser (31.1) umfasst, wobei der Durchmesser (43.1) des Diffusor-Grundkörpers (43) im Bereich der Einkoppelfläche (46) größer ist als oder gleich groß wie der Faserbündeldurchmesser (31.1) des Lichtleiters (30) im Bereich der Einkoppelfläche, wobei bevorzug das Verhältnis von Faserbündeldurchmesser (31.1) des Lichtleiters und Durchmesser des Diffusor-Grundkörpers (43) ≤ 1,0 bis 0,7, besonders bevorzugt von ≤ 1,0 bis 0,8 beträgt.

8. Beleuchtungssystem (1) nach mindestens einem der vorhergehenden Ansprüche, wobei das Diffusor-Element (40) am distalen Ende des Diffusor-Grundkörpers (43) eine gerichtet oder diffus reflektierende Reflektorfläche (47), die den Diffusor-Grundkörper (43) abschließt und/oder zumindest teilweise oder abschnittsweise an dessen Umfangsfläche umfasst, aufweist.

9. Beleuchtungssystem (1) nach mindestens einem der vorherigen Ansprüche, wobei die Reflektorfläche (47) vorzugsweise aus polierten, metallischen Drahtabschnitten ausgebildet ist, die direkt in Kontakt mit dem Diffusor-Grundkörper (43) gebracht sind,
und/oder die Reflektorfläche (47) als aufgesputterte oder aufgedampfte dielektrische Reflektionsschichten auf dem distalen Ende des Diffusor-Grundkörpers (43) ausgebildet sind, welche aus mehreren Schichten bestehen und hinsichtlich der Reflektivität auf die Wellenlänge des verwendeten Lichtes abgestimmt sind, wobei vorzugsweise ein Maximum der Reflektivität bei dieser Wellenlänge vorliegt und/oder
wobei die Reflektorfläche (47) bevorzugt als Silberschicht mit einer rückseitigen Passivierung ausgeführt ist.

10. Beleuchtungssystem (1) nach mindestens einem der vorhergehenden Ansprüche, wobei
die Reflektorfläche (47) konkav oder konvex und/ oder als direkt oder beabstandet, einen Hohlraum zwischen Reflektorfläche (47) und distalem Ende des Diffusor-Grundkörpers (43) bildend, an den Diffusor-Grundkörper (43) anschließender, Körper (47.1) und/ oder Hülle (47.2), als einseitig geschlossener, reflektierender Hohlkörper, ausgebildet ist.

11. Beleuchtungssystem (1) nach mindestens einem der vorhergehenden Ansprüche, wobei
sich zwischen dem proximalen Ende des Diffusor-Grundkörper (43) und dem distalen Ende des Lichtleiters (30) eine Verbindungszone (44) befindet, in der ein optisches Element und/ oder Zwischenmedium (45) angeordnet ist.

12. Beleuchtungssystem (1) nach Anspruch 11, wobei die Verbindungszone (44) vorzugsweise mit einem Abdeckmaterial, insbesondere einer Hülse (48), zumindest teilweise oder abschnittsweise abgedeckt ist.

13. Beleuchtungssystem (1) nach mindestens einem der vorhergehenden Ansprüche, wobei
der Diffusor-Grundkörper (43) mit der Reflektorfläche (47) und die Verbindungszone (44) zumindest teilweise oder abschnittsweise mit einer transparenten oder transluzenten, farblosen oder eingefärbten Hülle (49) umschlossen ist; bevorzugt die Hülle (49) aus einem starren Rohrabschnitt und/ oder aus einem flexiblen Schlauch gebildet ist, wobei der Rohrabschnitt und/ oder der Schlauch bevorzugt Streuzentren enthält.

14. Beleuchtungssystem (1) nach Anspruch 13, wobei die Hülle (49) zumindest abschnittsweise aus einem oder mehreren dünnwandigen Schrumpfschläuchen ausgeführt ist.

15. Beleuchtungssystem (1) nach mindestens einem der vorhergehenden Ansprüche, wobei
die Streuelemente (43.6)
- bei einer Glas-Matrix Poren, Partikel, poröse oder pigmentierten oder eingefärbte oder Inhomogenitäten beinhaltende Glas- oder Glaskeramik, oder Glaskeramik-Elementen und die darin umfassten Kristallite umfassen oder aus diesen bestehen,
- bei einer Quarz-Matrix Poren, poröses Quarzglas oder keramische oder polykristalline Partikel umfassen oder aus diesen bestehen
oder
- bei einer transparenten Glaskeramik-Matrix Poren, Partikel, poröse oder pigmentierte oder eingefärbte oder Inhomogenitäten beinhaltendes Glas oder Glaskeramik, oder Glaskeramik-Elemente und den darin umfassten Kristalliten umfassen oder aus diesen bestehen oder
- eine Kombination der jeweiligen Streuelemente (43.6) umfassen,
wobei
die Inhomogenitäten des Glases oder der Glaskeramik, die die Streuelemente (43.6) bei Glas- oder Glaskeramik-Matrixlösungen bilden, vorzugsweise Phasenseparation, Entmischungen und/oder partikuläre Einlagerung, Keime und/ oder Kristallite umfassen.

16. Beleuchtungssystem (1) nach zumindest einem der Ansprüche 14 oder 15, wobei
der Diffusor-Grundkörper (43) bevorzugt aus Borosilikat-, Phosphat-Kron-Glas, Blei-Silikat-Glas, Zinn-Silikat- oder Alkali-Zink-Glas und die Streuelemente (43.6) aus Weißglas-Stäbchen gebildet sind, die mit einem Hüllrohr (43.3) aus Borosilikatglas umschlossen sind.

17. Beleuchtungssystem (1), nach einem der vorhergehenden Ansprüche, wobei der Diffusor-Grundkörper (43) aus einer Mehrzahl von Abschnitten aus sich unterscheidenden Diffusor-Grundkörpern (43) nach mindestens einem der vorherigen Ansprüche gebildet ist.

18. Beleuchtungssystem (1), nach mindestens einem der vorherigen Ansprüche, wobei
die Streuelemente Streuzentren mit einer Streuzentrendichte pro Volumeneinheit aufweisen, wobei sich die Streuzentrendichte am proximalen Ende des Diffusor-Grundkörpers (43) von derjenigen am distalen Ende unterscheidet; bevorzugt ist die Streuzentrendichte am distalen Ende grösser als am proximalen Ende; besonders bevorzugt liegt ein Gradient der Streuzentrendichte vor und
wobei vorzugsweise
die Streuelemente (43.6) in unmittelbarer Nähe zu einer Einkoppelfläche (46) des Diffusor-Grundkörpers (43) und/ oder in unmittelbarer Nähe zur Reflektorfläche (47) eine gegenüber der Streuwirkung entlang des Diffusor-Grundkörpers (43) reduzierte Streuwirkung aufweisen.

19. Beleuchtungssystem (1), nach einem der vorherigen Ansprüche, wobei der Diffusor-Grundkörper (43) zumindest teilweise oder abschnittsweise kegelförmig ausgebildet ist.

20. Beleuchtungssystem (1), nach einem der vorhergehenden Ansprüche, wobei
das Diffusor-Element (40) und/ oder der Diffusor-Grundkörper (43) zumindest teilweise oder abschnittsweise in deren Volumen und/ oder auf deren Oberflächen strukturiert ist.

21. Beleuchtungssystem (1), nach einem der vorhergehenden Ansprüche, wobei der Diffusor-Grundkörper (43) eine Beschichtung aus streuenden Partikeln und/ oder der Diffusor-Grundkörper (43) eine zusätzliche Ummantelung aus einem eingefärbten Glas oder einem eingefärbten Kunststoff aufweist.

22. Beleuchtungssystem (1), nach einem der vorhergehenden Ansprüche, wobei
der Diffusor-Grundkörper (43) zumindest 10, bevorzugt zumindest 20 eingelagerte Streuelemente (43.6) und sich bevorzugt das Verhältnis der Querschnittsflächen von eingelagerten Streuelementen (43.6) und Diffusor-Grundkörper (43) zu ≤ 0,015, bevorzugt ≤ 0,005, besonders bevorzugt ≤ 0,002 ergibt.

23. Verfahren zum Herstellen eines Beleuchtungssystems (1), nach einem der vorhergehenden Ansprüche,
beinhaltend die Verfahrensschritte
- Bereitstellen einer Mehrzahl von Lichtleitstäben aus einem Glas mit dem Brechungsindex n₁ und/ oder n₁',
- Anordnen der Mehrzahl der Lichtleitstäbe mit dem Brechungsindex n₁ und/ oder n₁' und zumindest eines Streustabs aus einem Glas oder einer Glaskeramik, die Streuzentren umfaßt, so dass die Längsachsen der Lichtleitstäbe und des zumindest einen Streustabs zumindest im Wesentlichen parallel zueinander verlaufen und eine Vorform erhalten wird,
- Erwärmen der Vorform,
- Ausziehen der Vorform zu einem Diffusor-Grundkörper (43), so dass sich die Außenumfangsflächen der Lichtleitstäbe, unlösbar, formschlüssig miteinander und mit dem zumindest einem Streustab verbinden, insbesondere miteinander verschmelzen, und so die Matrix (43.4) des Diffusor-Grundkörpers (43) mit zumindest einem eingelagerten und/ oder an diesen angrenzendem Streuelement (43.4), gebildet aus dem zumindest einen ausgezogenen Streustab, bilden.

24. Verfahren nach Anspruch 23, wobei
das Erwärmen und Ausziehen der Vorform bei Temperaturen unterhalb der Schmelztemperatur des Glases der Mehrzahl der Lichtleitstäbe erfolgt, so dass
die Matrix des Diffusor-Grundkörpers (43) mit dem zumindest einen eingelagerten Streuelement (43.6) bevorzugt Phasengrenzen zwischen ausgezogenen Lichtleitstäben und/ oder Streustäben umfasst.

25. Verfahren nach zumindest einem der Ansprüche 23 oder 24, wobei der Diffusor-Grundkörpers (43) durch Variation der Parameter Geschwindigkeit, Temperatur und/ oder Kraft des Ziehprozesses der Vorform zumindest teilweise oder abschnittsweise sich verjüngend oder kegelförmig ausgebildet ist, so dass das zumindest eine Streuelement (43.6) im Bereich der Verjüngung einen Winkel zur Längsachse (43.2) des Diffusor-Grundkörpers (43) aufweisend verläuft.

26. Verfahren zum zumindest teilweise oder abschnittsweisen Strukturieren eines Beleuchtungssystems nach Anspruch 1, wobei der Diffusor-Grundkörper (43) mit einer Reflektorfläche (47) und einer Verbindungszone (44) zumindest teilweise oder abschnittsweise mit einer transparenten oder transluzenten Hülle (49) umschlossen ist und das Diffusor-Element (40) bildet; bevorzugt ist die Hülle (49) aus einem starren Rohrabschnitt und/ oder aus einem flexiblen Schlauch gebildet, bevorzugt enthält der Rohrabschnitt und/ oder der Schlauch Streuzentren,
die im Volumen und/ oder den Oberflächen dessen Eigenschaften und/oder Zusammensetzung zumindest lokal modifizieren und/oder materialab- oder auftragend Strukturen in nahezu beliebiger geometrischer Form und Anordnung in und/oder auf diesen ausbilden, umfassend
- Verfahren der Laserbearbeitung, insbesondere mittels Kurzpuls- oder CO₂-Lasern, die bevorzugt Brechwert- und/ oder
Zusammensetzungsänderung oder Erzeugen von Strukturen im Volumen und/ oder den Oberflächen einbringen
- Druckprozesse zum Aufbringen beziehungsweise Herstellen insbesondere einer Rasterverlaufsstruktur mittels druckbarer organischer oder keramischer Farben mit entsprechenden Pigmenten oder mittels einer Glasfluss-basierten Farben
- Verfahren des nasschemischen oder trockenchemischen Ätzens,
- fotolithografische Verfahren,
- abrasive, mechanische Bearbeitungsverfahren oder
eine Kombination dieser Verfahren.

## Claims

1. An illumination system (1) for a medical technology treatment and/or diagnosis system, comprising at least one laser light source (10) and an optical waveguide (30) which can be connected to the at least one laser light source (10) at a proximal end thereof, and comprising a diffuser element (40) at the distal end of the optical waveguide (30), the diffuser element having a longitudinal axis extending perpendicular to a coupling surface of the optical waveguide within the diffuser element (40); wherein
in its operating state, the diffuser element emits light over its active length (40.2) laterally of the longitudinal axis; wherein
the diffuser element (40) comprises at least one diffuser base body (43) and the diffuser base body (43) includes at least one scattering element (43.6);
wherein in the case of a consistent cross-sectional shape of the diffuser base body (43) the at least one scattering element (43.6) is aligned substantially parallel thereto along the entire longitudinal axis (43.2) thereof, or is arranged at an angle to the longitudinal axis in the case of tapering diffuser base bodies;
wherein means for homogenizing the emission intensity along the longitudinal axis (43.2) of the diffuser base body (43) are provided at the distal end of the diffuser base body (43) and/or surrounding the transition area between the optical waveguide (30) and the diffuser base body (43) and/or the diffuser base body (43) itself at least partially or in sections thereof, comprising sleeves, jackets, caps and/or layers, wherein the diffuser base body (43) comprises or consists of a matrix made of glass, fused silica, or transparent glass ceramics; and
wherein, in its operating state, the illumination system exhibits an intensity distribution of lateral emission deviating by not more than ± 50 %, preferably by not more than ± 30 %, and most preferably by not more than ± 5 % from the average lateral emission intensity.

2. The illumination system (1) according to claim 1, wherein
a plurality of scattering elements (43.6) is provided and arranged around the longitudinal axis of the diffuser element (40); wherein the scattering elements (43.6) are preferably arranged in a regular pattern around the longitudinal axis (43.2), in particular preferably in a circular pattern.

3. The illumination system (1) according to claim 2, wherein
the number of scattering elements (43.6) per unit area, based on the cross-sectional area of the diffuser base body (43), is greater outside a core zone (43.7) along the longitudinal axis (43.2) than within the core zone (43.7).

4. The illumination system (1) according to claim 1, wherein
the at least one scattering element (43.6) is tubular and in particular is arranged coaxially to the longitudinal axis (43.2) of the diffuser base body (43).

5. The illumination system (1) according to at least one of the preceding claims, wherein
the at least one scattering element (43.6) has a triangular, in particular hyperbolic triangular cross section, or a hexagonal, in particular hyperbolic hexagonal cross section.

6. The illumination system (1) according to at least one of the preceding claims, wherein
the diffuser base body (43) comprises a matrix (43.4) which has different refractive indices n₁ and n₁' with respect to the cross-sectional area thereof, in particular between the core zone (43.7) and the peripheral zone of the matrix (43.4) in which the at least one scattering element (43.6) is embedded.

7. The illumination system (1) according to claims 1 to 6, wherein
the optical waveguide (30) comprises a single fiber having a core (31) with a core diameter (31.1) and a cladding (32); wherein
the diameter (43.1) of the diffuser base body (43) in the area of the coupling surface is greater than or equal to the core diameter (31.1) of the optical waveguide (30) in the area of the coupling surface (46), wherein preferably the ratio of the core diameter (31.1) of the optical waveguide to the diameter of the diffuser base body (43) is between ≤ 1.0 and 0.7, most preferably between ≤ 1.0 and 0.8; or
wherein the optical waveguide comprises a fiber bundle (31) having a fiber bundle diameter (31.1), wherein the diameter (43.1) of the diffuser base body (43) in the area of the coupling surface (46) is greater than or equal to the fiber bundle diameter (31.1) of the optical waveguide (30) in the area of the coupling surface, wherein preferably the ratio of the fiber bundle diameter (31.1) of the optical waveguide to the diameter of the diffuser base body (43) is between ≤ 1.0 and 0.7, most preferably between ≤ 1.0 and 0.8.

8. The illumination system (1) according to at least one of the preceding claims, wherein at the distal end of the diffuser base body (43), the diffuser element (40) has a directionally or diffusely reflecting reflector surface (47) terminating the diffuser base body (43) and/or surrounding the lateral surface thereof at least partially or in sections thereof.

9. The illumination system (1) according to claim 8, wherein the reflector surface (47) is defined by polished metallic wire sections which are disposed in direct contact with the diffuser base body (43);
and/or wherein the reflector surface (47) is defined by sputter-deposited or vapor-deposited dielectric reflective layers on the distal end of the diffuser base body (43), consisting of multiple layers and matched to the wavelength of the employed light with regard to reflectivity, preferably by having a reflectivity maximum at this wavelength; and/or
wherein the reflector surface (47) is preferably implemented as a silver layer with rear passivation.

10. The illumination system (1) according to at least one of the preceding claims, wherein
the reflector surface (47) has a concave or convex shape and/or is in the form of a body (47.1) and/or a cover (47.2) directly adjoining the diffuser base body (43) or being spaced apart therefrom so as to define a cavity between the reflector surface (47) and the distal end of the diffuser base body (43) in the form of a reflective hollow body closed on one end.

11. The illumination system (1) according to at least one of the preceding claims, wherein
a conjunction zone (44) is provided between the proximal end of the diffuser base body (43) and the distal end of the optical waveguide (30), with an optical element and/or an intermediate medium (45) disposed therein.

12. The illumination system (1) according to claim 11, wherein
the conjunction zone (44) is covered by a covering material, in particular a sleeve (48), at least partially or in sections thereof.

13. The illumination system (1) according to at least one of the preceding claims, wherein
the diffuser base body (43) with the reflector surface (47) and the conjunction zone (44) are enclosed at least partially or in sections thereof by a transparent or translucent, colorless or dyed jacket (49); wherein the jacket (49) is preferably defined by a rigid tube section and/or by flexible tubing, wherein the tube section and/or tubing preferably include scattering centers.

14. The illumination system (1) according to claim 13, wherein the jacket (49) is at least partially made of one or more thin-walled heat-shrink tubes.

15. The illumination system (1) according to at least one of the preceding claims, wherein
the scattering elements (43.6) comprise or consist of
- pores, particles, porous or pigmented or dyed or inhomogeneities-containing glass or glass ceramic or glass ceramic elements and the crystallites contained therein in the case of a glass matrix;
- pores, porous fused silica, or ceramic or polycrystalline particles in the case of a fused silica matrix; or
- pores, particles, porous or pigmented or dyed or inhomogeneities-containing glass or glass ceramic or glass ceramic elements and the crystallites contained therein in the case of a transparent glass ceramic matrix; or
- a combination of the respective scattering elements (43.6);
wherein
the inhomogeneities of the glass or the glass ceramic, which define the scattering elements (43.6) in the case of glass or glass ceramic matrix implementations, preferably comprise phase separation, segregation and/or particulate incorporation, seeds and/or crystallites.

16. The illumination system (1) according to at least one of claims 14 or 15, wherein
the diffuser base body (43) is preferably made of borosilicate glass, phosphate crown glass, lead silicate glass, tin silicate glass, or alkali zinc glass, and the scattering elements (43.6) are defined by white glass rods which are enclosed by a cladding tube (43.3) made of borosilicate glass.

17. The illumination system (1) according to any one of the preceding claims, wherein the diffuser base body (43) is made of a plurality of portions consisting of differing diffuser base bodies (43) according to at least one of the preceding claims.

18. The illumination system (1) according to at least one of the preceding claims, wherein
the scattering elements comprise scattering centers with a scattering center density per unit volume, wherein the scattering center density at the proximal end of the diffuser base body (43) differs from that at the distal end; wherein, preferably, the scattering center density is greater at the distal end than at the proximal end; wherein most preferably the scattering center density has a gradient; and wherein
in the immediate vicinity of a coupling surface (46) of the diffuser base body (43) and/or in the immediate vicinity of the reflector surface (47), the scattering elements (43.6) preferably have a scattering effect that is reduced compared to the scattering effect along the diffuser base body (43).

19. The illumination system (1) according to any one of the preceding claims, wherein
the diffuser base body (43) has a conical shape at least partially or in sections thereof.

20. The illumination system (1) according to any one of the preceding claims, wherein
the diffuser element (40) and/or the diffuser base body (43) is structured within the volume and/or on the surface thereof, at least partially or in sections thereof.

21. The illumination system (1) according to any one of the preceding claims, wherein
the diffuser base body (43) has a coating of scattering particles; and/or wherein the diffuser base body (43) has an additional jacket made of a dyed glass or a dyed plastics material.

22. The illumination system (1), according to any one of the preceding claims, wherein
the diffuser base body (43) has at least 10, preferably at least 20 embedded scattering elements (43.6), and wherein preferably the resulting ratio of the cross-sectional area of embedded scattering elements (43.6) to that of the diffuser base body (43) is ≤ 0.015, preferably ≤ 0.005, most preferably ≤ 0.002.

23. A method for producing an illumination system (1) according to any one of the preceding claims, comprising the method steps of
- providing a plurality of fiber-optic rods made of a glass having a refractive index n₁ and/or n₁';
- arranging the plurality of fiber-optic rods having the refractive index n₁ and/or n₁' and at least one scattering rod made of a glass or a glass ceramic and comprising scattering centers, in such a way that the longitudinal axes of the fiber-optic rods and of the at least one scattering rod extend at least substantially parallel to one another to obtain a preform;
- heating said preform;
- drawing the preform to form a diffuser base body (43) such that the outer circumferential surfaces of the fiber-optic rods inseparably bond to one another and to the at least one scattering rod to form a positive fit, in particular so as to fuse to one another, and so as to form the matrix (43.4) of the diffuser base body (43) with at least one embedded and/or adjoining scattering element (43.4) formed from the at least one drawn scattering rod.

24. The method according to claim 23, wherein
the heating and drawing of the preform is performed at temperatures below the melting temperature of the glass of the majority of the fiber-optic rods; so that the matrix of the diffuser base body (43) including the at least one embedded scattering element (43.6) preferably comprises phase boundaries between drawn fiber-optic rods and/or scattering rods.

25. The method according to at least one of claims 23 or 24, wherein the diffuser base body (43) is formed into a tapering or conical shape at least partially or in sections thereof by varying the parameters of rate, temperature, and/or force of the drawing process of the preform, so that within the range of the taper the at least one scattering element (43.6) extends at an angle relative to the longitudinal axis (43.2) of the diffuser base body (43).

26. A method for structuring an illumination system according to claim 1 at least partially or in sections thereof,
wherein the diffuser base body (43) with a reflector surface (47) and a conjunction zone (44) is at least partially or in sections thereof enclosed by a transparent or translucent jacket (49) to form the diffuser element (40); wherein, preferably, a jacket (49) is defined by a rigid tube section and/or by flexible tubing, wherein the tube section and/or tubing preferably contains scattering centers
which modify the properties and/or composition thereof in the volume and/or at the surfaces at least locally, and/or form structures of virtually any desired geometrical shape and arrangement therein and/or thereon by material removing or material depositing techniques comprising
- laser processing techniques, in particular using short-pulse or CO₂ lasers, which preferably introduce modifications in refractive index and/or composition or create structures within the volume and/or at the surfaces;
- printing techniques for applying or producing in particular a grid structure using printable organic or ceramic inks which contain appropriate pigments, or using a glass flux-based ink;
- wet chemical or dry chemical etching techniques;
- photolithographic processes;
- abrasive mechanical processing techniques; or
a combination of these techniques.

## Revendications

1. Système d'éclairage (1) destiné à un système technique médical de thérapie et/ou de diagnostic, comprenant au moins une source de lumière laser (10) et un guide de lumière (30) qui peut être raccordé par une extrémité proximale à la source de lumière laser (10), au nombre d'au moins une, et présentant, à l'extrémité distale du guide de lumière (30), un élément diffuseur (40) avec un axe longitudinal qui s'étend perpendiculairement à une surface de couplage du guide de lumière dans l'élément diffuseur (40), dans lequel
l'élément diffuseur (40), à l'état de fonctionnement, émet de la lumière sur sa longueur active (40.2), latéralement par rapport à l'axe longitudinal, sachant que
l'élément diffuseur (40) présente au moins un corps de base de diffuseur (43), et le corps de base de diffuseur (43) contient au moins un élément diffuseur (43.6),
sachant que
l'élément diffuseur (43.6), au nombre d'au moins un, est orienté le long de l'axe longitudinal (43.2) complet du corps de base de diffuseur (43) à section uniforme, de manière sensiblement parallèle à celui-ci, ou est disposé en formant un angle avec l'axe longitudinal, lorsque les corps de base de diffuseur diminuent en section,
sachant que
à l'extrémité distale du corps de base de diffuseur (43) et/ou dans la zone de transition entre le guide de lumière (30) et le corps de base de diffuseur (43) et/ou, entourant le corps de base de diffuseur (43) au moins en partie ou par portions, il est prévu des dispositifs destinés à l'homogénéisation de l'intensité de rayonnement le long de l'axe longitudinal (43.2) du corps de base de diffuseur (43), comprenant des manchons, des gaines, des capuchons et/ou des couches, le corps de base de diffuseur (43) comprenant une matrice (43.4) en verre, verre de quartz ou vitrocéramique transparente, ou étant constitué de celle-ci, et
sachant que le système d'éclairage, à l'état de fonctionnement, présente une répartition d'intensité du rayonnement latéral qui s'écarte de l'intensité de rayonnement latérale moyenne d'au maximum ± 50 %, de préférence d'au maximum ± 30 %, et de manière nettement préférée d'au maximum ± 5 %.

2. Système d'éclairage (1) selon la revendication 1, dans lequel il est prévu une pluralité d'éléments diffuseurs (43.6) qui sont disposés autour de l'axe longitudinal de l'élément diffuseur (40), et les éléments diffuseurs (43.6) sont de préférence disposés en une structure régulière autour de l'axe longitudinal (43.2), notamment de préférence en forme de cercle.

3. Système d'éclairage (1) selon la revendication 2, dans lequel le nombre d'éléments diffuseurs (43.6) par unité de surface, rapporté à l'aire de section transversale du corps de base de diffuseur (43), est plus élevé à l'extérieur d'une zone de noyau (43.7), le long de l'axe longitudinal (43.2), que dans la zone de noyau (43.7).

4. Système d'éclairage (1) selon la revendication 1, dans lequel l'élément diffuseur (43.6), au nombre d'au moins un, est disposé sous une forme tubulaire et notamment de manière coaxiale à l'axe longitudinal (43.2) du corps de base de diffuseur (43).

5. Système d'éclairage (1) selon au moins une des revendications précédentes, dans lequel l'élément diffuseur (43.6), au nombre d'au moins un, présente une section triangulaire, notamment triangulaire ou hexagonale hyperbolique, et de manière particulièrement avantageuse une section hexagonale hyperbolique.

6. Système d'éclairage (1) selon au moins une des revendications précédentes, dans lequel le corps de base de diffuseur (43), rapporté à son aire de section transversale, présente une matrice (43.4) qui a des indices de réfraction n₁ et n₁' différents, notamment entre la zone de noyau (43.7) et la zone périphérique de la matrice (43.4) dans laquelle est incorporé l'élément diffuseur (43.6), au nombre d'au moins un.

7. Système d'éclairage (1) selon les revendications 1 à 6, dans lequel le guide de lumière (30) comprend une fibre unique comportant un cœur (31), avec un diamètre de cœur (31.1), et une gaine (32), où le diamètre (43.1) du corps de base de diffuseur (43), dans la région de la surface de couplage, est supérieur ou égal au diamètre de cœur (31.1) du guide de lumière (30) dans la région de la surface de couplage (46),
où le rapport entre le diamètre de cœur (31.1) du guide de lumière et le diamètre du corps de base de diffuseur (43) est de préférence ≤ 1,0 à 0,7, de manière particulièrement avantageuse ≤ 1,0 à 0,8, ou
le guide de lumière comprend un faisceau de fibres (31) présentant un diamètre de faisceau de fibres (31.1), où le diamètre (43.1) du corps de base de diffuseur (43), dans la région de la surface de couplage (46), est supérieur ou égal au diamètre de faisceau de fibres (31.1) du guide de lumière (30) dans la région de la surface de couplage, où le rapport entre le diamètre de faisceau de fibres (31.1) du guide de lumière et le diamètre du corps de base de diffuseur (43) est de préférence ≤ 1,0 à 0,7, de manière particulièrement avantageuse ≤ 1,0 à 0,8.

8. Système d'éclairage (1) selon au moins une des revendications précédentes, dans lequel l'élément diffuseur (40) présente, à l'extrémité distale du corps de base de diffuseur (43), une surface de réflecteur (47) à réflexion dirigée ou diffuse qui termine le corps de base de diffuseur (43) et/ou entoure celui-ci au moins en partie ou par portions sur sa surface périphérique.

9. Système d'éclairage (1) selon au moins une des revendications précédentes, dans lequel la surface de réflecteur (47) est réalisée de préférence à partir de portions de fil métallique polies qui sont amenées directement en contact avec le corps de base de diffuseur (43),
et/ou la surface de réflecteur (47) est réalisée sous forme de couches de réflexion diélectriques déposées par pulvérisation cathodique ou en phase vapeur sur l'extrémité distale du corps de base de diffuseur (43), lesquelles couches sont constituées de plusieurs couches et sont adaptées en termes de réflectivité à la longueur d'onde de la lumière utilisée, un maximum de la réflectivité étant de préférence présent pour cette longueur d'onde, et/ou
dans lequel la surface de réflecteur (47) est réalisée de préférence sous forme de couche d'argent avec une passivation sur la face arrière.

10. Système d'éclairage (1) selon au moins une des revendications précédentes, dans lequel la surface de réflecteur (47) est réalisée de manière concave ou convexe et/ou comme corps (47.1) et/ou enveloppe (47.2) se raccordant au corps de base de diffuseur (43) directement ou en étant espacé(e), en formant une cavité entre la surface de réflecteur (47) et l'extrémité distale du corps de base de diffuseur (43), sous forme de corps creux réfléchissant, fermé sur un côté.

11. Système d'éclairage (1) selon au moins une des revendications précédentes, dans lequel il est prévu, entre l'extrémité proximale du corps de base de diffuseur (43) et l'extrémité distale du guide de lumière (30), une zone de liaison (44) dans laquelle est disposé un élément optique et/ou un milieu intermédiaire (45).

12. Système d'éclairage (1) selon la revendication 11, dans lequel la zone de liaison (44) est recouverte de préférence avec un matériau de recouvrement, notamment un manchon (48), au moins en partie ou par portions

13. Système d'éclairage (1) selon au moins une des revendications précédentes, dans lequel le corps de base de diffuseur (43), avec la surface de réflecteur (47), et la zone de liaison (44) sont entourés au moins partiellement ou par portions d'une gaine (49) transparente ou translucide, incolore ou teintée, la gaine (49) étant de préférence formée d'un tronçon de tube rigide et/ou d'un tuyau souple, le tronçon de tube et/ou le tuyau contenant de préférence des centres de diffusion.

14. Système d'éclairage (1) selon la revendication 13, dans lequel la gaine (49) est réalisée au moins par portions à partir d'une ou plusieurs gaines thermorétractables à paroi mince.

15. Système d'éclairage (1) selon au moins une des revendications précédentes, dans lequel
les éléments diffuseurs (43.6)
- avec une matrice de verre, comportent des pores, des particules, du verre ou de la vitrocéramique poreux ou pigmentés ou colorés ou contenant des inhomogénéités, ou des éléments en vitrocéramique et les cristallites contenus dans ceux-ci, ou sont constitués de ceux-ci,
- avec une matrice de quartz, comportent du verre de quartz poreux ou des particules céramiques ou polycristallines, ou sont constitués de ceux-ci,
ou
- avec une matrice de vitrocéramique transparente, comportent des pores, des particules, du verre ou de la vitrocéramique poreux ou pigmentés ou colorés ou contenant des inhomogénéités, ou des éléments en vitrocéramique et les cristallites contenus dans ceux-ci, ou sont constitués de ceux-ci,
sachant que
les inhomogénéités du verre ou de la vitrocéramique, qui forment les éléments diffuseurs (43.6) avec des solutions à matrice de verre ou de vitrocéramique, comprennent de préférence la séparation de phase, des ségrégations et/ou l'inclusion particulaire, des germes et/ou des cristallites.

16. Système d'éclairage (1) selon au moins une des revendications 14 ou 15, dans lequel le corps de base de diffuseur (43) est de préférence constitué de verre borosilicate, verre phosphate, verre phosphate à couronne, verre de silicate de plomb, verre de silicate d'étain ou verre alcalin au zinc, et les éléments diffuseurs (43.6) sont constitués de bâtonnets de verre blanc qui sont entourés d'un tube de gainage (43.3) en verre borosilicate.

17. Système d'éclairage (1) selon l'une des revendications précédentes, dans lequel le corps de base de diffuseur (43) est formé d'une pluralité de portions de corps de base de diffuseur (43) différents les uns des autres, selon au moins une des revendications précédentes.

18. Système d'éclairage (1) selon au moins une des revendications précédentes, dans lequel les éléments diffuseurs présentent des centres de diffusion avec une densité de centres de diffusion par unité de volume, où la densité de centres de diffusion à l'extrémité proximale du corps de base de diffuseur (43) se distingue de celle à l'extrémité distale ; de préférence, la densité de centres de diffusion à l'extrémité distale est supérieure à celle de l'extrémité proximale, et de manière particulièrement avantageuse, il existe un gradient de la densité de centres de diffusion, et dans lequel
les éléments diffuseurs (43.6), à proximité immédiate d'une surface de couplage (46) du corps de base de diffuseur (43) et/ou à proximité immédiate de la surface de réflecteur (47), présentent de préférence un effet de diffusion réduit par rapport à l'effet de diffusion le long du corps de base de diffuseur (43).

19. Système d'éclairage (1) selon l'une des revendications précédentes, dans lequel le corps de base de diffuseur (43) est réalisé au moins en partie ou par portions en forme de cône.

20. Système d'éclairage (1) selon l'une des revendications précédentes, dans lequel l'élément diffuseur (40) et/ou le corps de base de diffuseur (43) sont structurés au moins partiellement ou par portions dans leur volume et/ou à leurs surfaces.

21. Système d'éclairage (1) selon l'une des revendications précédentes, dans lequel le corps de base de diffuseur (43) présente un revêtement formé de particules diffusantes et/ou le corps de base de diffuseur (43) présente une enveloppe supplémentaire constituée d'un verre coloré ou d'une matière synthétique colorée.

22. Système d'éclairage (1) selon l'une des revendications précédentes, dans lequel le corps de base de diffuseur (43) au moins 10, de préférence au moins 20 éléments diffuseurs (43.6) incorporés, et le rapport entre les aires de section transversale d'éléments diffuseurs (43.6) incorporés et le corps de base de diffuseur (43) est de préférence ≤ 0,015, notamment ≤ 0,005, et de manière particulièrement avantageuse ≤ 0,002.

23. Procédé de fabrication d'un système d'éclairage (1) selon l'une des revendications précédentes, comprenant les étapes de procédé suivantes :
- mise à disposition d'une pluralité de tiges guides de lumière faites d'un verre présentant l'indice de réfraction n₁ et/ou n₁',
- mise en place de la pluralité de tiges guides de lumière présentant l'indice de réfraction n₁ et/ou n₁', et d'au moins une tige de diffusion constituée d'un verre ou d'une vitrocéramique qui comporte des centres de diffusion, de sorte que les axes longitudinaux des tiges guides de lumière et de la tige de diffusion, au nombre d'au moins une, s'étendent au moins sensiblement de manière parallèle les uns aux autres, et que l'on obtient une préforme,
- chauffage de la préforme,
- étirage de la préforme pour obtenir un corps de base de diffuseur (43), de sorte que les surfaces périphériques extérieures des tiges guides de lumière se lient de manière indissociable, avec complémentarité de forme, entre elles et avec la tige de diffusion, au nombre d'au moins une, notamment par fusion, et forment ainsi la matrice (43.4) du corps de base de diffuseur (43), avec au moins un élément diffuseur (43.4) qui est incorporé et/ou adjacent à celui-ci et constitué de la tige de diffusion étirée, au nombre d'au moins une.

24. Procédé selon la revendication 23, selon lequel le chauffage et l'étirage de la préforme sont réalisés à des températures inférieures à la température de fusion du verre de la pluralité de tiges guides de lumière, de manière à ce que la matrice du corps de base de diffuseur (43) comporte avec l'élément diffuseur (43.6) incorporé, au nombre d'au moins un, de préférence des limites de phase entre des tiges guides de lumière étirées et/ou les tiges de diffusion.

25. Procédé selon au moins l'une des revendications 23 ou 24, selon lequel le corps de base de diffuseur (43) est réalisé au moins en partie ou par portions en diminuant de section ou en formant un cône, par variation des paramètres que sont la vitesse, la température et/ou la force du processus d'étirage de la préforme, de sorte que l'élément diffuseur (43.6), au nombre d'au moins un, s'étend en présentant, dans la région de la diminution de section, un angle par rapport à l'axe longitudinal (43.2) du corps de base de diffuseur (43).

26. Procédé de structuration, au moins partielle ou par portions, d'un système d'éclairage selon la revendication 1, selon lequel le corps de base de diffuseur (43), avec une surface de réflecteur (47) et une zone de liaison (44), est entouré au moins en partie ou par portions d'une gaine (49) transparente ou translucide et forme l'élément diffuseur (40) ; de préférence, une gaine (49) est formée d'un tronçon de tube rigide et/ou d'un tuyau souple, le tronçon de tube et/ou le tuyau contenant de préférence des centres de diffusion qui, dans le volume et/ou les surfaces, modifient au moins localement les propriétés et/ou la composition de celui-ci et/ou forment dans et/ou sur ceux-ci, par enlèvement ou dépôt de matière, des structures ayant quasiment n'importe quelles formes et configurations,
comprenant
- des procédés de traitement laser, notamment avec des lasers à impulsions courtes ou des lasers CO₂, qui engendrent de préférence une modification de l'indice de réfraction et/ou de la composition, ou la production de structures dans le volume et/ou les surfaces,
- processus d'impression destinés à appliquer ou réaliser notamment une structure à tracé de trame, à l'aide de peintures organiques ou céramiques imprimables, avec des pigments correspondants, ou à l'aide d'une peinture à base de flux de verre,
- procédés de gravure chimique par voie humide ou gravure chimique sèche,
- procédés photolithographiques,
- procédés de traitement mécaniques abrasifs ou
une combinaison de ces procédés.
